# EUROPÄISCHE PATENTSCHRIFT

(11) **EP 0 292 822 B1**
(45) Veröffentlichungstag und Bekanntmachung des Hinweises auf die Patenterteilung: **12.01.1994**
(21) Anmeldenummer: 88107802.6
(22) Anmeldetag: 16.05.1988
(51) Int. Cl.: C07D 401/06, C07D 417/06, C07D 277/32, C07D 413/06, C07D 403/06, A01N 43/50, A01N 43/78, A01N 43/76, A01N 43/82, A01N 43/74, A01N 43/54

(54) **Substituierte Nitroalkene**
Substituted nitroalkenes
Nitroalkènes substitués

(30) Priorität: 27.05.1987 DE 3717837
(43) Veröffentlichungstag der Anmeldung: 30.11.1988
(73) Patentinhaber: BAYER AG, 51368 Leverkusen (DE)
(72) Erfinder: Wolf, Hilmar, Dr., D-4018 Langenfeld (DE); Becker, Benedikt, Dr., D-4020 Mettmann (DE); Stendel, Wilhelm, Dr., D-5600 Wuppertal 1 (DE); Homeyer, Bernhard, Dr., D-5090 Leverkussen 3 (DE)

(56) Entgegenhaltungen:
- EP-A- 0 154 178
- EP-A- 0 163 855
- EP-A- 0 192 060
- EP-A- 0 212 600
- DE-A- 2 624 530
- US-A- 3 962 234
- US-A- 3 993 648
- US-A- 4 029 791
- US-A- 4 053 619

## Beschreibung

Die vorliegende Erfindung betrifft neue substituierte Nitroalkene, ein Verfahren zu ihrer Herstellung und ihre Verwendung in Schädlingsbekämpfungsmitteln, insbesondere als Insektizide.

Es ist bereits bekannt geworden, daß bestimmte organische Nitroverbindungen, wie z. B. 2-Nitromethylen-2H-tetrahydro-1,3-thiazin, insektizide Eigenschaften aufweisen (vgl. US-PS 3 993 648).

Aus EP-OS 192 060 sind Insektizide, z.B. 1-(2-Chloro-5-pyridylmethyl)-2 [(4-chlorphenylthio)nitromethylen]-imidazolidin bekannt.

Es wurden nun die neuen substituierten Nitroalkene der allgemeinen Formel (I) 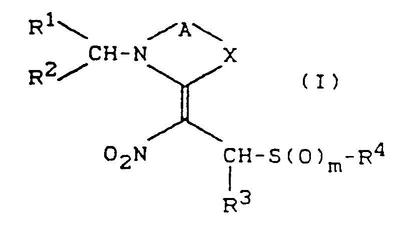
in welcher
- m: für die Zahlen 0, 1 oder 2 steht,
- A: für Alkandiyl steht,
- R¹: für eine fünf- oder sechsgliedrige heterocyclische Gruppierung steht, welche 1, 2, 3 oder 4 Stickstoffatome und/oder ein oder zwei Sauerstoff- oder Schwefelatome als Heteroatom-Ringglieder enthält - wobei die Zahl der Heteroatome 1, 2, 3 oder 4 beträgt - und welche gegebenenfalls durch Halogen, Cyano, Nitro, Alkyl, Halogenalkyl, Alkenyl, Halogenalkenyl, Alkinyl, Alkoxy, Halogenalkoxy, Alkenyloxy, Halogenalkenyloxy, Alkinyloxy, Alkylthio, Halogenalkythio, Alkenylthio, Halogenalkenylthio, Alkinylthio, Alkylsulfinyl, Halogenalkylsulfinyl, Alkylsulfonyl, Halogenalkylsulfonyl, Amino, Alkylamino, Dialkylamino, Aryl, Aryloxy, Arylthio, Arylamino, Aralkyl, Formylamino, Alkylcarbonylamino, Formyl, Carbamoyl, Alkylcarbonyl und/oder Alkoxycarbonyl substituiert ist,
- R²: für Wasserstoff oder Alkyl steht,
- R³: für Wasserstoff, Alkyl (welches gegebenenfalls durch Halogen, Cyano, Alkoxy oder Alkylthio substituiert ist), Alkenyl (welches gegebenenfalls durch Halogen oder Phenyl substituiert ist), Alkinyl, Phenyl (welches gegebenenfalls durch Halogen, Cyano, Nitro, Alkyl, Halogenalkyl, Alkoxy, Halogenalkoxy, Alkylthio, Halogenalkylthio, Alkylsulfinyl, Alkylsulfonyl, Halogenalkylsulfonyl, Dialkylamino oder Alkoxycarbonyl substituiert ist), Benzyl (welches gegebenenfalls durch Halogen, Cyano, Nitro, Alkyl, Halogenalkyl oder Alkoxycarbonyl substituiert ist), Furyl, Furylmethyl, Thenyl, Thienyl oder Pyridyl (welche gegebenenfalls durch Halogen oder Alkyl substituiert sind) steht,
- R⁴: für Phenyl (welches gegebenenfalls durch Halogen, Cyano, Nitro, Alkyl, Halogenalkyl, Alkoxy, Halogenalkoxy, Alkylthio, Halogenalkylthio, Amino, Alkylamino, Dialkylamino oder Alkoxycarbonyl substituiert ist), Naphthyl (welches gegebenenfalls durch Halogen, Cyano, Nitro, Alkyl oder Amino substituiert ist), Pyridinyl (welches gegebenenfalls durch Halogen oder Alkyl substituiert ist), Pyrimidinyl (welches gegebenenfalls durch Halogen oder Alkyl substituiert ist), Imidazolyl oder Triazolyl steht und
- X: für Sauerstoff, Schwefel oder die Gruppierung N-R⁵ steht, worin
- R⁵: für Wasserstoff, Alkyl (welches gegebenenfalls durch Halogen, Cyano, Alkoxy, Alkylthio, Dialkylamino, Trialkylsilyl, Alkoxycarbonyl, Carboxy, Carbamoyl, Alkylaminocarbonyl, Dialkylaminocarbonyl oder durch den Rest R¹ substituiert ist, wobei R¹ die oben angegebene Bedeutung besitzt), für Alkenyl (welches gegebenenfalls durch Halogen substituiert ist), Alkinyl, Benzyl (welches gegebenenfalls durch Halogen, Cyano, Nitro, Alkyl, Halogenalkyl, Alkoxy oder Alkoxycarbonyl substituiert ist), Formyl, Alkylcarbonyl (welches gegebenenfalls durch Halogen, Cyano, Phenyl, Phenoxy oder Alkoxy substituiert ist), Cycloalkylcarbonyl (welches gegebenenfalls durch Halogen und/oder Alkyl substituiert ist), Alkenylcarbonyl (welches gegebenenfalls durch Halogen substituiert ist), Phenylcarbonyl oder Naphthylcarbonyl (welche gegebenenfalls durch Halogen, Alkyl, Halogenalkyl, Cyano, Nitro, Alkoxy und/oder Alkoxycarbonyl substituiert sind), Alkoxycarbonyl, Benzyloxycarbonyl, Phenoxycarbonyl, Alkylthiocarbonyl, Benzylthiocarbonyl, Phenylthiocarbonyl, Alkylaminocarbonyl, Dialkylaminocarbonyl, Phenylaminocarbonyl (welches gegebenenfalls durch Halogen, Cyano, Nitro, Alkyl, Halogenalkyl, Alkoxy, Halogenalkoxy, Alkylthio, Halogenalkylthio oder Alkoxycarbonyl substituiert ist), Benzoylaminocarbonyl (welches gegebenenfalls durch Halogen, Alkyl oder Halogenalkyl substituiert ist), Phenylsulfonylaminocarbonyl (welches gegebenenfalls durch Halogen, Alkyl, Halogenalkyl, Alkoxy, Halogenalkoxy oder Alkoxycarbonyl substituiert ist), Alkylthio (welches gegebenenfalls durch Halogen substituiert ist), Phenylthio (welches gegebenenfalls durch Halogen, Nitro oder Alkyl substituiert ist), Alkylsulfinyl, Alkylsulfonyl (welches gegebenenfalls durch Halogen substituiert ist), Phenylsulfinyl (welches gebenenfalls durch Halogen, Nitro oder Alkyl substituiert ist), Phenylsulfonyl oder Naphthylsulfonyl (welche gegebenenfalls durch Halogen, Cyano, Nitro, Alkyl, Halogenalkyl, Alkoxy, Halogenalkoxy und/oder Alkoxycarbonyl substituiert sind), Dialkyl(thio)phosphoryl, Alkyl-alkoxy-(thio)phosphoryl oder Dialkoxy(thio)phosphoryl steht,
gefunden.

Die vorliegende Erfindung betrifft auch die einzelnen möglichen Z- und E-Isomeren der obigen Formel (I) und der nachstehenden Formel (I′) - worin m, A, R¹, R², R³, R⁴ und X die oben angegebenen Bedeutungen haben - und die möglichen optischen Isomeren - soweit R² und/oder R³ von Wasserstoff verschieden sind - sowie beliebige Gemische aller möglichen Isomeren.

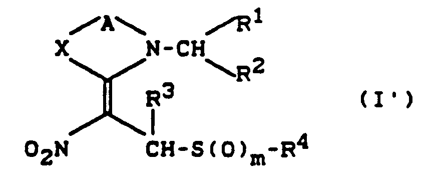

Im folgenden bezieht sich die vereinfachende Formulierung "Verbindungen der Formel (I)" jeweils auf die durch Formel (I) und (I′) skizzierten Verbindungen.

Man erhält die neuen substituierten Nitroalkene der allgemeinen Formel (I), wenn man Nitroalkene der allgemeinnen Formel (II) 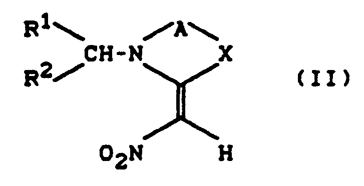
in welcher
- A, R¹, R² und X: die oben angegebenen Bedeutungen haben,
- bzw. die Isomeren der korrespondierenden Konfiguration (Z bzw. E) bzw. Gemische der Z- und E-Isomeren -
mit Aldehyden der allgemeinen Formel (III) 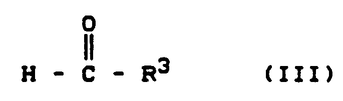
in welcher
- R³: die oben angegebenen Bedeutung hat,
und mit Mercaptoverbindungen der allgemeinen Formel (IV)

HS - R⁴ (IV)

in welcher
- R⁴: die oben angegebene Bedeutung hat,
gegebenenfalls in Gegenwart einer Säure und gegebenenfalls in Gegenwart eines Verdünnungsmittels umsetzt und gegebenenfalls die so erhaltenen Verbindungen der Formel (I), in welcher m für die Zahl 0 steht und A, R¹, R², R³, R⁴ und X die oben angegebenen Bedeutungen haben, nach üblichen Oxidationsmethoden in entsprechende Verbindungen der Formel (I), in welcher dann m für die Zahlen 1 oder 2 steht und A, R¹, R², R³, R⁴ und X die oben angegebenen Bedeutungen haben, umwandelt.

Weitere mögliche Herstellungsmethoden für die erfindungsgemäßen Verbindungen der allgemeinen Formel (I) sind nachstehend schematisch aufgeführt, wobei m, R¹, R², R³, R⁴ und X die oben angegebenen Bedeutungen haben:
(a) Umsetzung von Halogenverbindungen der Formel (V) (Y = Halogen) mit Nitroalkenen der Formel (VI), gebenenfalls in Gegenwart eines Säureakzeptors, wie z. B. Kaliumcarbonat, und gegebenenfalls in Gegenwart eines Verdünnungsmittels, wie z. B. Aceton, Acetonitril oder Dimethylformamid, bei Temperaturen zwischen 10 °C und 100 °C: 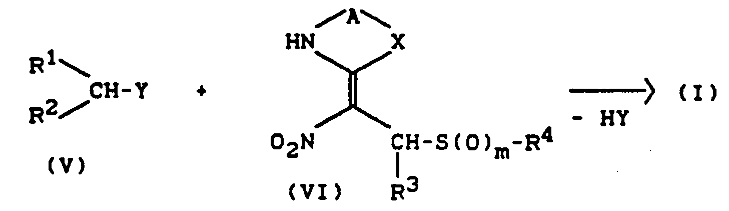
(b) Umsetzung von Nitroalkenen der Formel (IA) (X = NH) mit Halogenverbindungen der Formel (VII) (Z = Halogen), gegebenenfalls in Gegenwart eines Säureakzeptors, wie z. B. Kaliumcarbonat, und gegebenenfalls in Gegenwart eines Verdünnungsmittels, wie z. B. Aceton, Acetonitril oder Dimethylformamid, bei Temperaturen zwischen 10 °C und 100 °C: 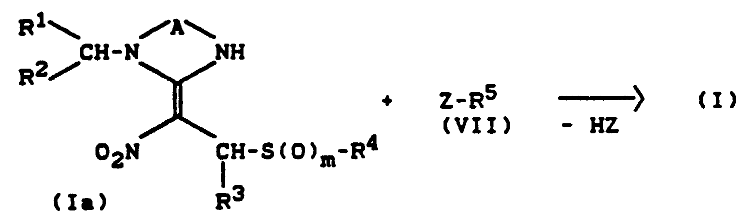
(c) Umsetzung von Aminen der Formel (VIII) mit 1,1-Bismethylthio-3-nitro-alkenen der Formel (IX), gegebenenfalls in Gegenwart eines Verdünnungsmittels, wie z. B. Toluol, Xylol oder Dioxan, bei Temperaturen zwischen 10 °C und 150 °C:

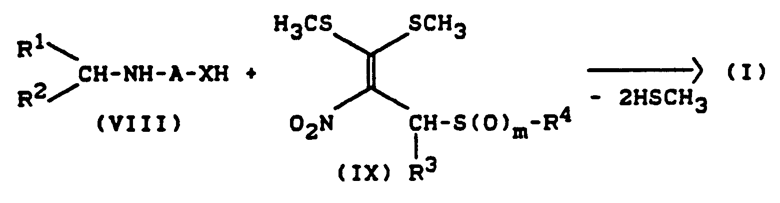

Die neuen substituierten Nitroalkene der allgemeinen Formel (I) zeichnen sich durch hohe Wirksamkeit als Insektizide aus. Überraschenderweise zeigen der erfindungsgemäßen Verbindungen der Formel (I) erheblich stärkere insektizide Wirkung als nach Struktur und Wirkprofil vergleichbare organische Nitroverbindungen, wie z. B. 2-Nitromethylen-2H-tetrahydro-1,3-thiazin.

Die Erfindung betrifft vorzugsweise Verbindungen der Formel (I), in welcher
- m: für die Zahlen 0, 1 oder 2 steht,
- A: für C₂-C₅-Alkandiyl steht,
- R¹: für eine fünf- bzw. sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4,-1,2,5- oder 1,3,4-Thiadiazolyl, Pyridyl, Pyridazinyl, Pyrimidinyl und Pyrazinyl steht, welcher gegebenenfalls durch Fluor, Chlor, Brom, Iod, Cyano, Nitro, C₁-C₄-Alkyl (welches gegebenenfalls durch Fluor und/oder Chlor substituiert ist), C₂-C₄-Alkenyl (welches gegebenenfalls durch Fluor und/oder Chlor substituiert ist), C₂-C₄-Alkinyl, C₁-C₄-Alkoxy (welches gegebenenfalls durch Fluor und/oder Chlor substituiert ist), C₃-C₄-Alkenyloxy (welches gegebenenfalls durch Fluor und/oder Chlor substituiert ist), C₃-C₄-Alkinyloxy, C₁-C₄-Alkylthio (welches gegebenenfalls durch Fluor und/oder Chlor substituiert ist), C₃-C₄-Alkenylthio (welches gegebenenfalls durch Fluor und/oder Chlor substituiert ist), C₃-C₄-Alkinylthio, C₁-C₄-Alkylsulfinyl (welches gegebenenfalls durch Fluor und/oder Chlor substituiert ist), C₁-C₄-Alkylsulfonyl (welches gegebenenfalls durch Fluor und/oder Chlor substituiert ist), Amino, C₁-C₄-Alkylamino, Di-(C₁-C₄-alkyl)-amino, Phenyl, Phenoxy, Phenylthio, Phenylamino, Benzyl, Formylamino, C₁-C₄-Alkyl-carbonylamino, Formyl, Carbamoyl, C₁-C₄-Alkyl-carbonyl und/oder C₁-C₄-Alkoxy-carbonyl substituiert ist,
- R²: für Wasserstoff oder C₁-C₃-Alkyl steht,
- R³: für Wasserstoff, C₁-C₄-Alkyl (welches gegebenenfalls durch Fluor, Chlor, Cyano, C₁-C₄-Alkoxy oder C₁-C₄-Alkylthio substituiert ist), C₂-C₄-Alkenyl (welches gegebenenfalls durch Fluor, Chlor oder Phenyl substituiert ist), C₂-C₄-Alkinyl, Phenyl (welches gegebenenfalls durch Fluor, Chlor, Brom, Cyano, Nitro, C₁-C₄-Alkyl, Trifluoromethyl, C₁-C₄-Alkoxy, C₁-C₂-Fluoralkoxy, C₁-C₂-Chlorfluoralkoxy, C₁-C₄-Alkylthio, C₁-C₂-Fluoralkylthio, C₁-C₂-Chlorfluoralkylthio, C₁-C₄-Alkylsulfinyl, C₁-C₄-Alkylsulfonyl, C₁-C₂-Fluoralkylsulfonyl, C₁-C₂-Chlorfluoralkylsulfonyl, Di-(C₁-C₃-alkyl)-amino oder C₁-C₃-Alkoxy-carbonyl substituiert ist), Benzyl (welches gegebenenfalls durch Fluor, Chlor, Cyano, Nitro, C₁-C₂-Alkyl, Trifluormethyl oder C₁-C₃-Alkoxy-carbonyl substituiert ist), Furyl, Furylmethyl, Thenyl, Thienyl oder Pyridyl (welche gegebenenfalls durch Fluor, Chlor oder C₁-C₄-Alkyl substituiert sind) steht,
- R⁴: für Phenyl (welches gegebenenfalls durch Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist, C₁-C₄-Alkylthio (welches gegebenenfalls durch Fluor und/oder Chlor substituiert ist), Amino, C₁-C₄-Alkylamino, Di-(C₁-C₄-alkyl)-amino oder C₁-C₄-Alkoxycarbonyl substituiert ist), Naphthyl (welches gegebenenfalls durch Chlor, Cyano, Nitro, Amino oder C₁-C₄-Alkyl substituiert ist), Pyridinyl (welches gegebenenfalls durch Fluor, Chlor, Brom oder C₁-C₄-Alkyl substituiert ist), Pyrimidinyl (welches gegebenenfalls durch Fluor, Chlor oder C₁-C₄-Alkyl substituiert ist), Imidazolyl oder Triazolyl steht und
- X: für Sauerstoff, Schwefel oder die Gruppierung N-R⁵ steht, worin
- R⁵: für Wasserstoff, C₁-C₄-Alkyl (welches gegebenenfalls durch Fluor, Chlor, Cyano, C₁-C₄-Alkoxy, C₁-C₄-Alkylthio, Di-(C₁-C₄-alkyl)-amino, Trimethylsilyl, C₁-C₄-Alkoxy-carbonyl, Carboxy, Carbamoyl, C₁-C₄-Alkyl-aminocarbonyl, Di-(C₁-C₃-alkyl)-aminocarbonyl, oder durch eine heterocyclische Gruppierung, wie sie oben für R¹ (einschließlich der möglichen Substituenten) vorzugsweise definiert ist, substituiert ist), C₂-C₄-Alkenyl (welches gegebenenfalls durch Fluor oder Chlor substituiert ist), C₂-C₄-Alkinyl, Benzyl (welches gegebenenfalls durch Fluor, Chlor, Cyano, Nitro, C₁-C₂-Alkyl, Trifluormethyl, C₁-C₂-Alkoxy oder C₁-C₂-Alkoxy-carbonyl substituiert ist), Formyl, C₁-C₂₀-Alkyl-carbonyl (welches gegebenenfalls durch Fluor, Chlor, Brom, Cyano, Phenyl, Phenoxy oder C₁-C₄-Alkoxy substituiert ist), C₃-C₆-Cycloalkyl-carbonyl (welches gegebenenfalls durch Fluor, Chlor und/oder C₁-C₄-Alkyl substituiert ist), C₂-C₂₀-Alkenyl-carbonyl (welches gegebenenfalls durch Fluor und/oder Chlor substituiert ist), Phenylcarbonyl oder Naphthylcarbonyl (welche gegebenenfalls durch Fluor, Chlor, Brom, C₁-C₄-Alkyl, Trifluormethyl, Cyano, Nitro, C₁-C₄-Alkoxy und/oder C₁-C₄-Alkoxy-carbonyl substituiert sind), C₁-C₂₀-Alkoxy-carbonyl, Benzyloxy-carbonyl, Phenoxycarbonyl, C₁-C₄-Alkylthio-carbonyl, Benzylthio-carbonyl, Phenylthio-carbonyl, C₁-C₆-Alkylamino-carbonyl, Di-(C₁-C₄-alkyl)-amino-carbonyl, Phenylamino-carbonyl (welches gegebenenfalls durch Fluor, Chlor, Brom, Cyano, Nitro, C₁-C₄-Alkyl, Trifluormethyl, C₁-C₄-Alkoxy, C₁-C₂-Fluoralkoxy, C₁-C₂-Chlor-fluoralkoxy, C₁-C₄-Alkylthio, C₁-C₂-Fluoralkylthio, C₁-C₂-Chlorfluoralkylthio oder C₁-C₄-Alkoxycarbonyl substituiert ist), Benzoylamino-carbonyl (welches gegebenenfalls durch Fluor, Chlor, Brom, Methyl oder Trifluormethyl substituiert ist), Phenylsulfonylamino-carbonyl (welches gegebenenfalls durch Fluor, Chlor, Brom, Methyl, Trifluormethyl, C₁-C₄-Alkoxy, C₁-C₂-Fluoralkoxy, C₁-C₂-Chlorfluoralkoxy oder C₁-C₄-Alkoxy-carbonyl substituiert ist), C₁-C₄-Alkylthio (welches gegebenenfalls durch Fluor und/oder Chlor substituiert ist), Phenylthio (welches gegebenenfalls durch Fluor, Chlor, Brom, Nitro oder Methyl substituiert ist), C₁-C₄-Alkylsulfinyl, C₁-C₄-Alkylsulfonyl (welches gegebenenfalls durch Fluor und/oder Chlor substituiert ist), Phenylsulfinyl (welches gegebenenfalls durch Fluor, Chlor, Brom, Nitro oder Methyl substituiert ist) Phenylsulfonyl oder Naphthylsulfonyl (welche gegebenenfalls durch Fluor, Chlor, Brom, Cyano, Nitro, Methyl, Trifluormethyl, C₁-C₄-Alkoxy, C₁-C₂-Fluoralkoxy, C₁-C₂-Chlorfluoralkoxy und/oder C₁-C₄-Alkoxy-carbonyl substituiert sind), Dimethyl(thio)phosphoryl, C₁-C₄-Alkyl-C₁-C₄-alkoxy-(thio)phosphoryl oder Di-(C₁-C₄-alkoxy)(thio)phosphoryl steht.

Die Erfindung betrifft insbesondere Verbindungen der Formel (I), in welcher
- m: für die Zahlen 0, 1 oder 2 steht,
- A: für Ethan-1,2-diyl (Dimethylen) oder Propan-1,3-diyl (Trimethylen) steht,
- R¹: für eine fünf- bzw. sechsgliedrige heterocyclische Gruppierung aus der Reihe Pyrazolyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl steht, welche gegebenenfalls durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert ist,
- R²: für Wasserstoff steht,
- R³: für Wasserstoff steht,
- R⁴: für Phenyl (welches gegebenenfalls durch Fluor, Chlor, C₁-C₄-Alkyl, Trifluormethyl, C₁-C₂-Alkoxy (welches gegebenenfalls durch Fluor und/oder Chlor substituiert ist) oder C₁-C₂-Alkoxy-carbonyl substituiert ist) oder für Naphthyl steht und
- X: für Schwefel oder die Gruppierung N-R⁵ steht, worin
- R⁵: für Wasserstoff, Methyl, Ethyl, Allyl, Propargyl, Formyl, C₁-C₈-Alkyl-carbonyl, Phenylcarbonyl (welches gegebenenfalls durch Fluor und/oder Chlor substituiert ist), C₁-C₈-Alkoxy-carbonyl, Benzyloxy-carbonyl, Phenoxy-carbonyl, Benzyl (welches gegebenenfalls durch Fluor oder Chlor substituiert ist) oder Di-(C₁-C₂-alkoxy)-(thio)phosphoryl steht.

Verwendet man zur Durchführung des erfindungsgemäßen Herstellungsverfahrens für die Verbindungen der allgemeinen Formel (I) als Ausgangsstoffe beispielsweise 1-(2-Chlor-thiazol-5-yl-methyl)-2-nitroimino-imidazolidin, Acetaldehyd und Thiophenol, so kann die Reaktion dieser Verbindungen durch das folgende Formelschema skizziert werden:

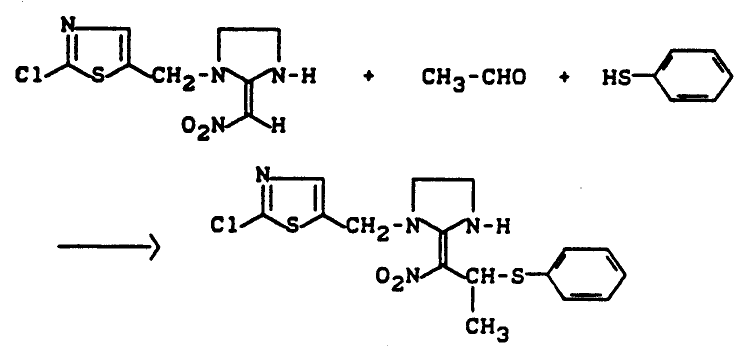

Die beim erfindungsgemäßen Herstellungsverfahren als Ausgangsstoffe zu verwendenden Nitroalkene sind durch die Formel (II) allgemein definiert. In dieser Formel (II) haben A, R¹, R² und X vorzugsweise bzw. insbesondere die gleichen Bedeutungen, wie sie bereits im Zusammenhang mit der Beschreibung der erfindungsgemäßen Verbindungen der Formel (I) vorzugsweise bzw. als insbesondere bevorzugt genannt wurden.

Beispiele für die Ausgangsstoffe der Formel (II) sind in der nachstehenden Tabelle 1 aufgeführt.

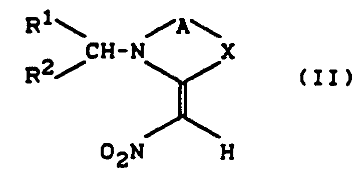

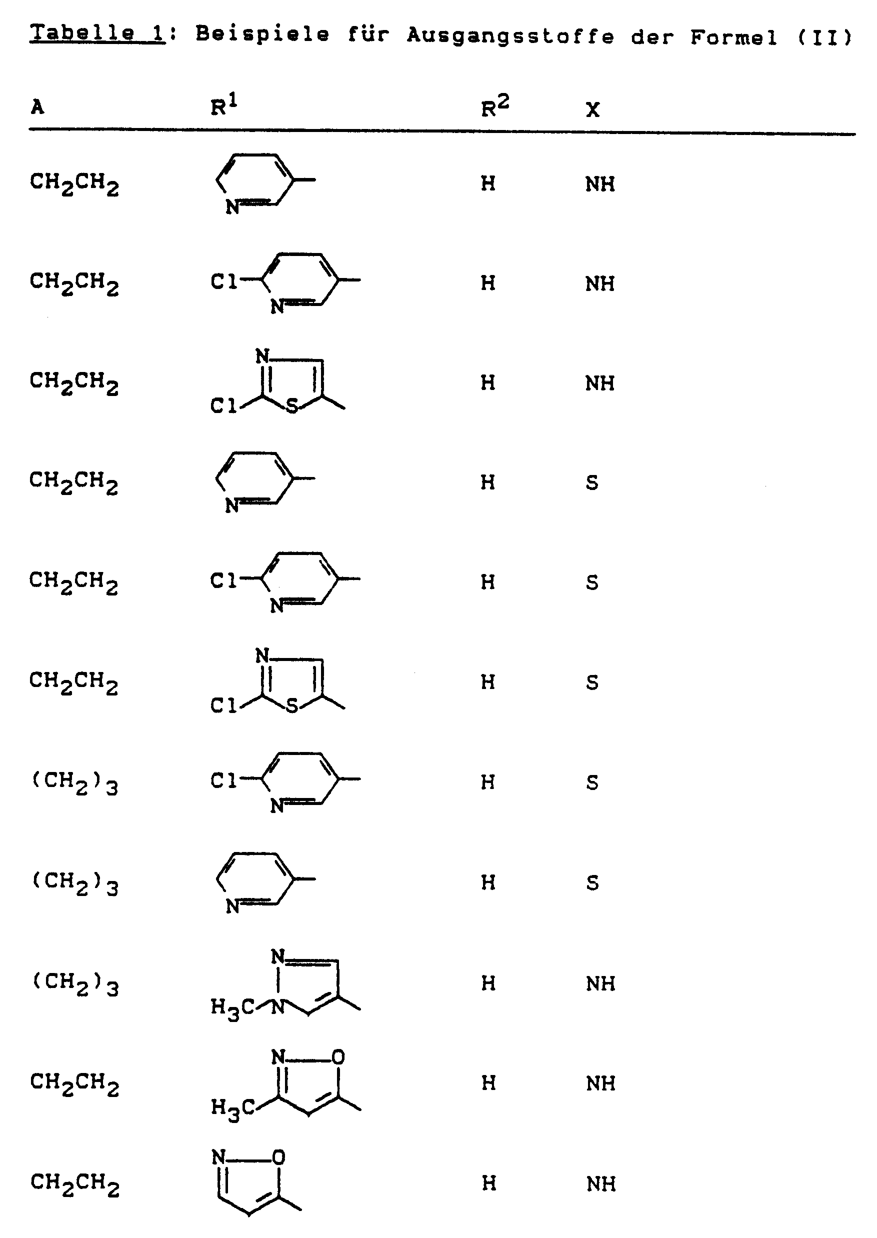

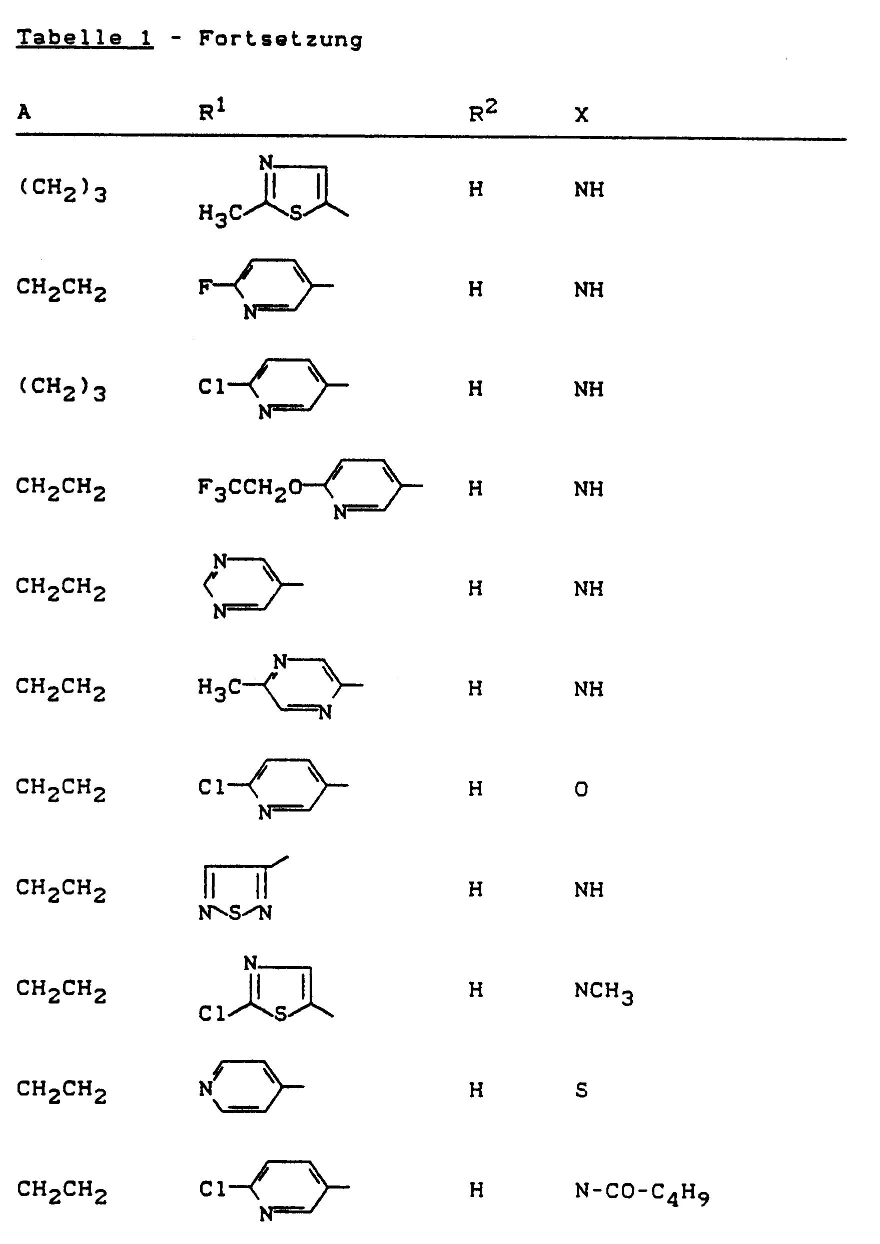

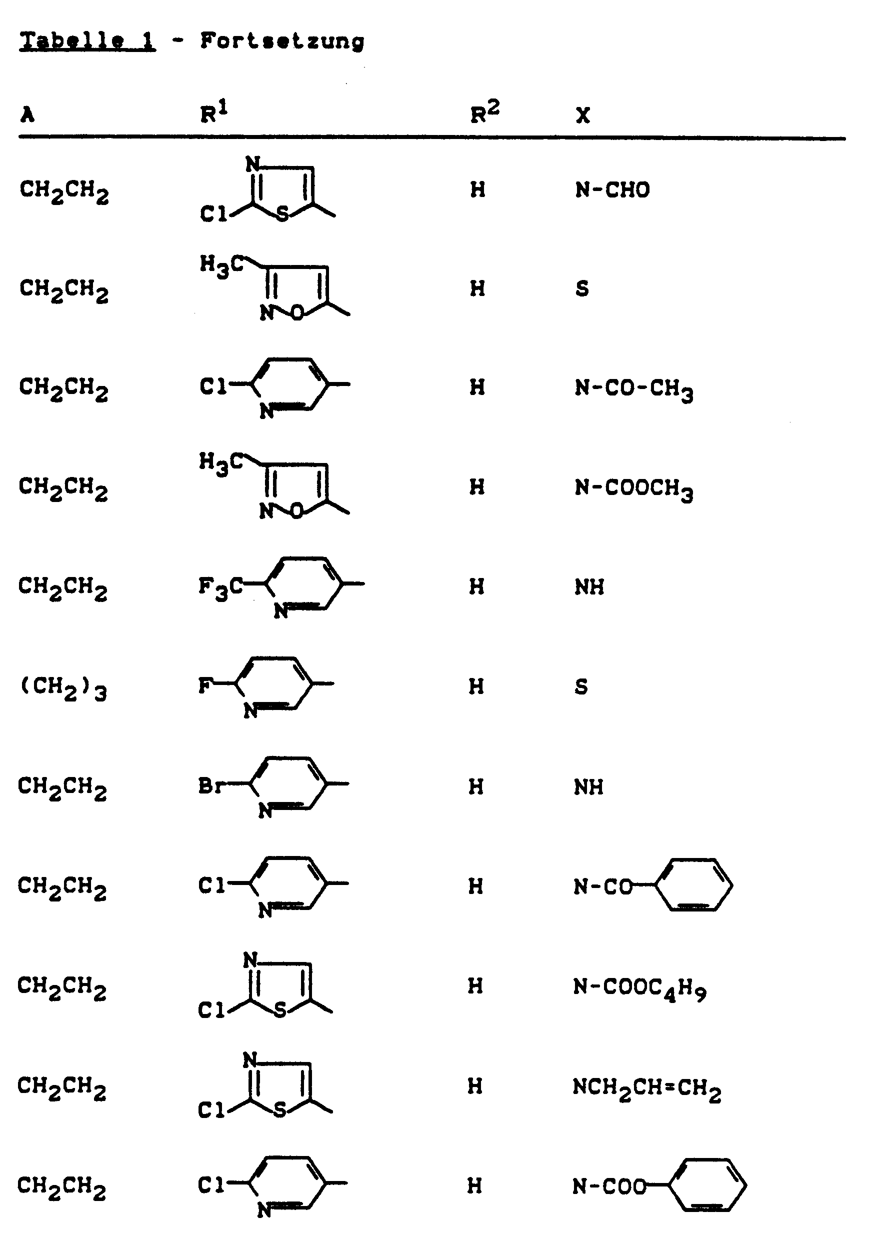

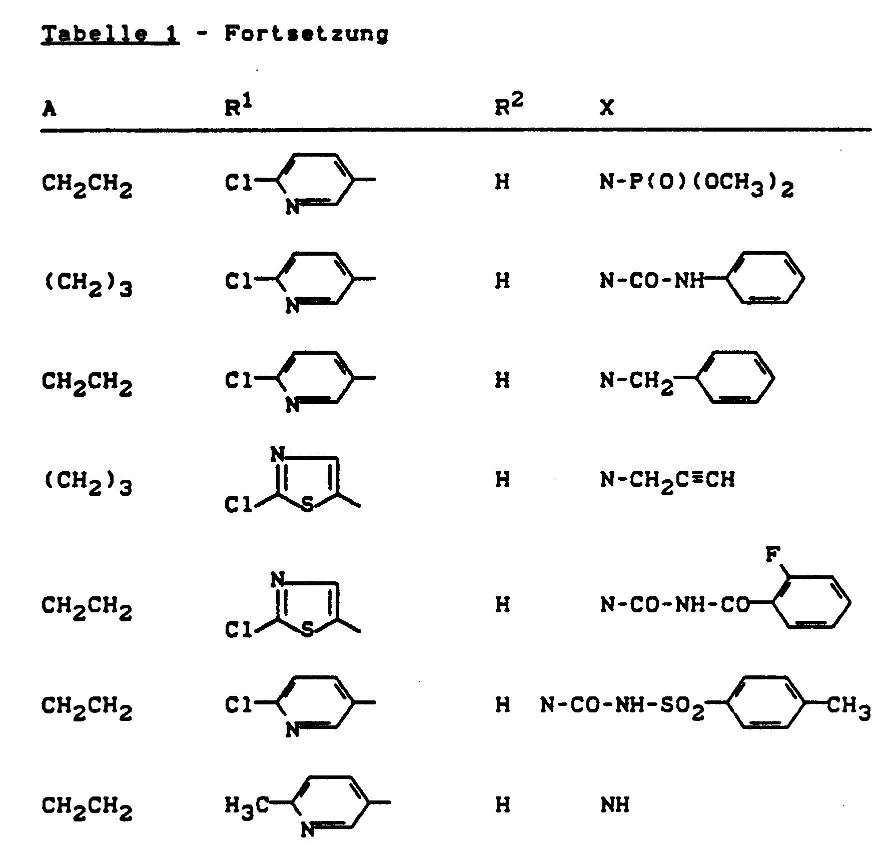

Die Ausgangsstoffe der Formel (II) sind bekannt und/oder können nach an sich bekannten Verfahren hergestellt werden (vgl. EP-A 192 060).

Die weiter als Ausgangsstoffe zu verwendenden Aldehyde sind durch die Formel (III) allgemein definiert. In dieser Formel (III) hat R³ vorzugsweise bzw. insbesondere die gleiche Bedeutung, wie sie bereits im Zusammenhang mit der Beschreibung der erfindungsgemäßen Vebindungen der Formel (I) vorzugsweise bzw. als insbesondere bevorzugt angegeben ist.

Als Beispiele für die Ausgangsstoffe der Formel (III) seien genannt:
Formaldehyd, Acetaldehyd, Propionaldehyd, Butyraldehyd, Chloracetaldehyd, Dichloracetaldehyd, Trichloracetaldehyd, Crotonaldehyd, Zimtaldehyd, Benzaldehyd, Phenylacetaldehyd, Furfural und Pyridin-2-, -3- und -4-carbaldehyd.

Die Ausgangsstoffe der Formel (III) sind bekannte Chemikalien.

Die weiter als Ausgangsstoffe zu verwendenden Mercaptoverbindungen sind durch die Formel (IV) allgemein definiert. In dieser Formel (IV) hat R⁴ vorzugsweise bzw. insbesondere die gleiche Bedeutung, wie sie bereits im Zusammenhang mit der Beschreibung der erfindungsgemäßen Verbindungen der Formel (I) vorzugsweise bzw. als insbesondere bevorzugt angegeben ist.

Als Beispiele für die Ausgangsstoffe der Formel (IV) seien genannt:
Thiophenol, 2-Chlor-, 3-Chlor- und 4-Chlor-thiophenol, 2-Fluor-, 3-Fluor- und 4-Fluor-thiophenol, 2-Brom-, 3-Brom- und 4-Brom-thiophenol, 2,3-Dichlor-, 2,4-Dichlor-, 2,5-Dichlor-, 2,6-Dichlor-, 3,4-Dichlor- und 3,5-Dichlor-thiophenol, 4-Methyl-, 4-Ethyl-, 4-Propyl-, 4-Isopropyl-, 4-Butyl-, 4-Isobutyl-, 4-sec-Butyl- und 4-tert-Butyl-thiophenol, 3-Methoxy- und 4-Methoxy-thiophenol, 2-Methoxycarbonyl-, 2-Ethoxycarbonyl-, 4-Methoxycarbonyl- und 4-Ethoxycarbonyl-thiophenol sowie Naphthalin-1-thiol und Naphthalin-2-thiol.

Die Ausganggstoffe der Formel (IV) sind bekannte Chemikalien.

Das erfindungsgemäße Verfahren zur Herstellung der neuen substituierten Nitroalkene der Formel (I) wird vorzugsweise unter Verwendung von Verdünnungsmitteln durchgeführt. Als Verdünnungsmittel kommen insbesondere protisch-polare Solventien, wie Wasser, Alkohole (wie z. B. Methanol, Ethanol, Propanol, Isopropanol, Butanol, Isobutanol und tert-Butanol) und Alkandiole oder Derivate (wie z. B. Ethan-1,2-diol und 2-Methoxyethanol) sowie Gemische dieser Solventien, aber auch weniger polare Lösungsmittel (wie z. B. Benzol, Toluol, Dioxan und Nitrobenzol) in Betracht.

Das erfindungsgemäße Verfahren wird gegebenenfalls in Gegenwart von Säuren durchgeführt. Als solche kommen vorzugsweise Protonensäuren, wie z. B. Salzsäure, Schwefelsäure, Phosphorsäure, Essigsäure, Methansulfonsäure, Benzolsulfonsäure, p-Toluolsulfonsäure und Naphthalin-1- oder -2-sulfonsäure, in Betracht.

Die Reaktionstemperaturen können bei dem erfindungsgemäßen Verfahren in einem größeren Bereich variiert werden. Im allgemeinen arbeitet man bei Temperaturen zwischen 0 °C und 120 °C, vorzugsweise bei Temperaturen zwischen 10 °C und 100 °C.

Das erfindungsgemäße Verfahren wird im allgemeinen unter Normaldruck oder geringfügig erhöhtem Druck durchgeführt.

Zur Durchführung des erfindungsgemäßen Verfahrens setzt man im allgemeinen je Mol Ausgangsverbindung der Formel (II), 1,0 bis 1,2 Mol, vorzugsweise 1,0 bis 1,05 Mol, Aldehyd der Formel (III) und 1,0 bis 1,5 Mol, vorzugsweise 1,0 bis 1,2 Mol, Mercaptoverbindung der Formel (IV) ein.

Im allgemeinen wird der Ausgangsstoff der Formel (II) bei Raumtemperatur mit dem Lösungsmittel und dann nacheinander mit der Mercaptoverbindung der Formel (IV) und dem Aldehyd der Formel (III) vermischt und das Reaktionsgemisch wird anschließend, gegebenenfalls bei erhöhter Temperatur, bis zum Ende der Umsetzung gerührt und/oder unter Rückfluß zum Sieden erhitzt.
Die Produkte der Formel (I) fallen beim Abkühlen im allgemein kristallin an und können durch Absaugen isoliert werden.

Die so erhaltenen Verbindungen der Formel (I) - m = 0 - können nach üblichen Oxidationsmethoden in entsprechende Sulfoxide - m = 1 - oder Sulfone - m = 2 - umgewandelt werden (vgl. Tetrahedron 42 (1986), 5459 - 5495), beispielsweise durch Umsetzung mit Wasserstoffperoxid in Gegenwart von Selendioxid in Methanol/Wasser (vgl. Synthesis 1978, 758 - 759), in Gegenwart von Titan(III)-chlorid in Methylenchlorid/Wasser (vgl. Synthesis 1981, 204 - 205), in Gegenwart von Natriumwolframat in Wasser (vgl. EP-A 137 417) oder durch Umsetzung mit Alkalimetallhypochloriten in Wasser (vgl. EP-A 125 654) bei Temperaturen zwischen 0 °C und 50 °C.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus Der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Bevircoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Lactrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sind durch hervorragende insektizide Wirksamkeit aus. Sie zeigen insbesondere beim Einsatz als Blattinsektizide und Bodeninsektizide hervorragende Wirkung, z. B. gegen Käferlarven (z. B. Phaedon cochleariae), gegen Schmetterlingsraupen (z. B. Spodoptera frugiperda) und gegen Blattläuse (z. B. Myzus persicae).

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sog. Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskülar, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw..

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel- Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Triebgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

### Herstellungsbeispiele

### Beispiel 1

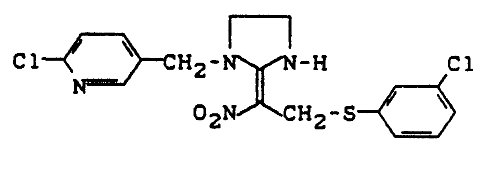

10 ml einer 35-%igen wässrigen Formaldehyd-Lösung (entsprechend 0,12 Mol) werden bei 25 °C unter Rühren zu einer Mischung aus 25,4 g (0,10 Mol) 1-(2-Chlor-pyridin-5-yl-methyl)-2-nitromethylen-imidazolidin, 14,4 g (0,10 Mol) 3-Chlor-thiophenol und 500 ml Ethanol tropfenweise gegeben und das Reaktionsgemisch wird anschließend fünf Stunden unter Rückfluß zum Sieden erhitzt. Nach dem Abkühlen auf 20 °C wird das kristallin angefallene Produkt durch Absaugen isoliert.

Man erhält 31 g (69 % der Theorie) 1-(2-Chlor-pyridin-5-yl-methyl)-2-(1-nitro-2-(3-chlor-phenylthio)-ethyliden)-imidazolidin vom Schmelzpunkt 143 °C.

Analog Beispiel 1 und entsprechend der allgemeinen Beschreibung des erfindungsgemäßen Herstellungsverfahrens können die in der nachstehenden Tabelle 2 aufgeführten Verbindungen der allgemeinen Formel (I) hergestellt werden.

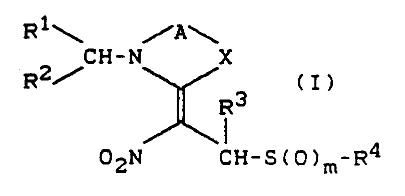

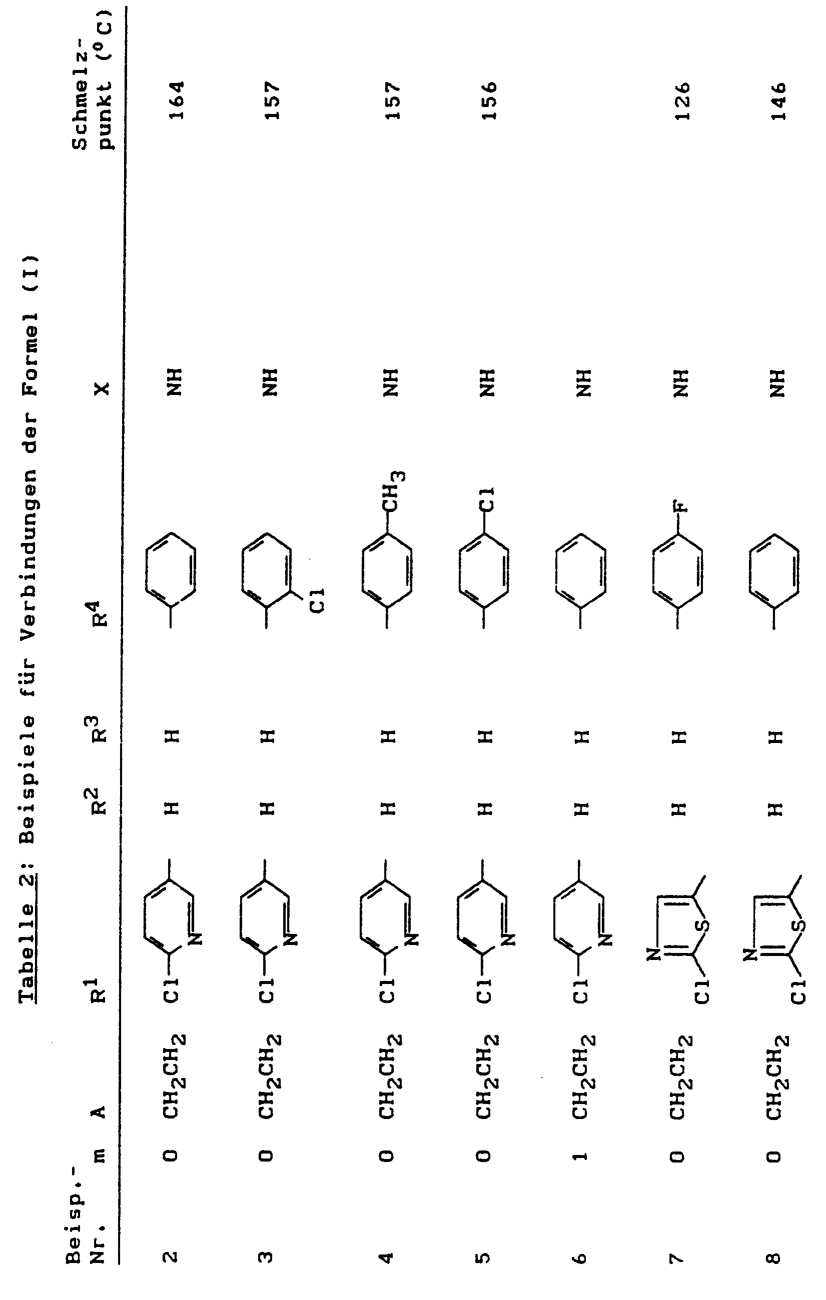

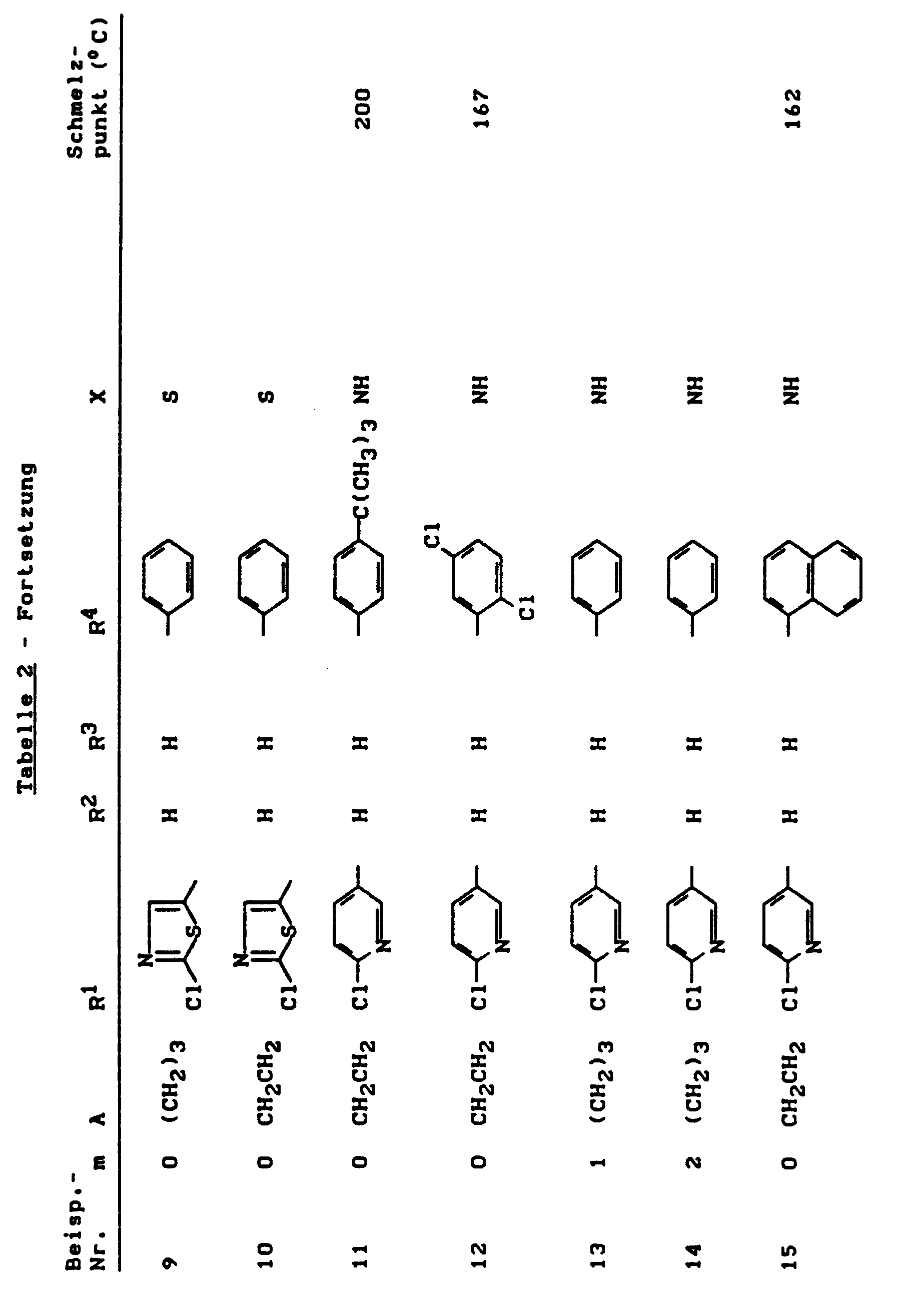

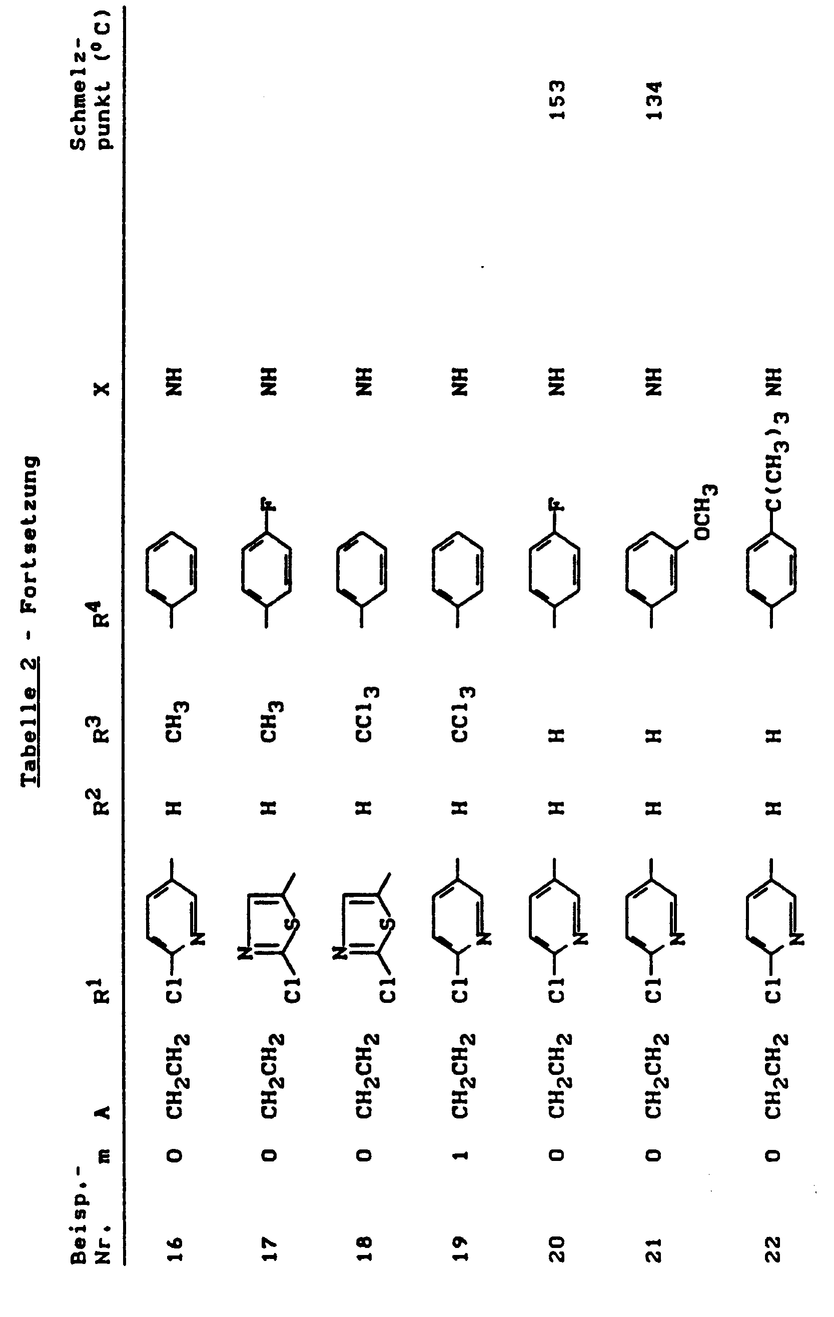

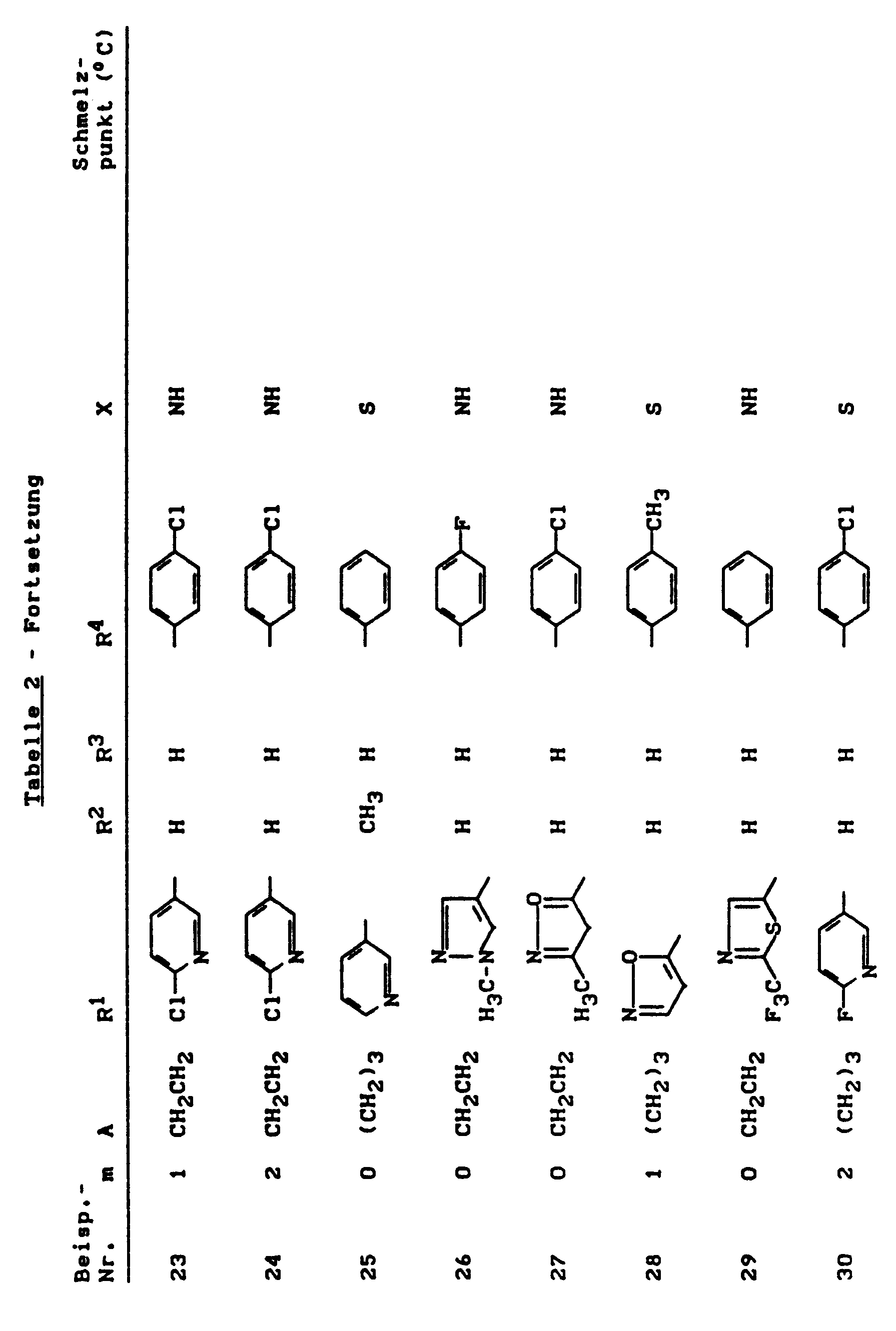

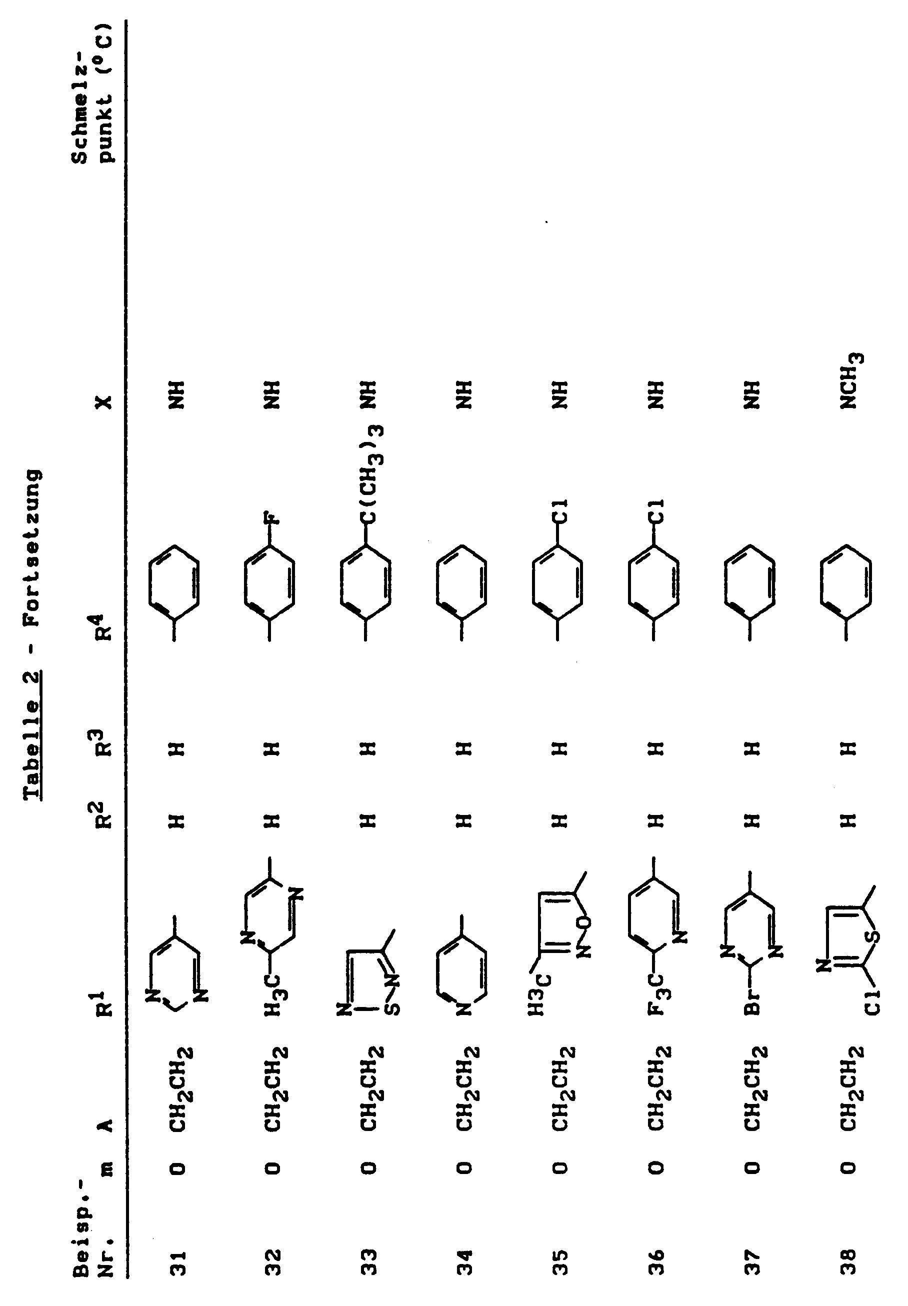

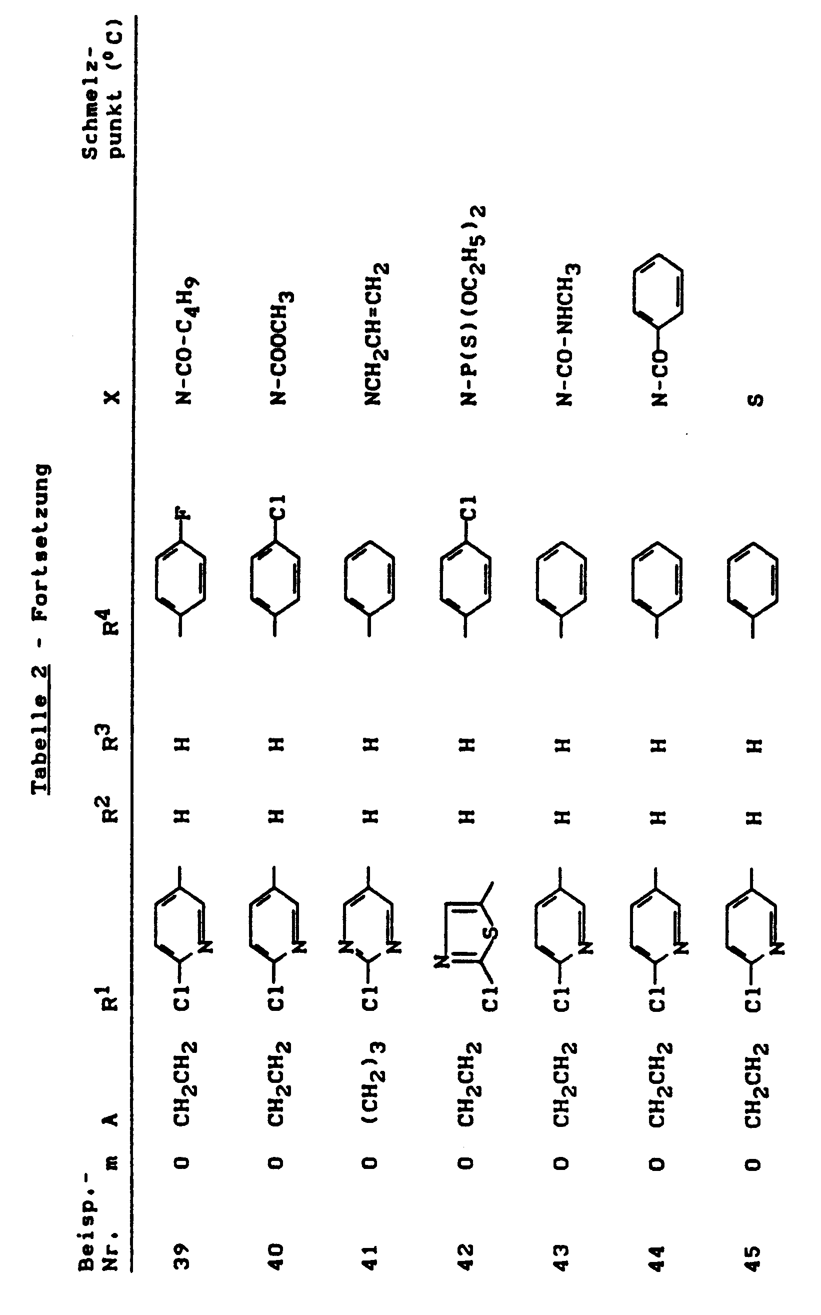

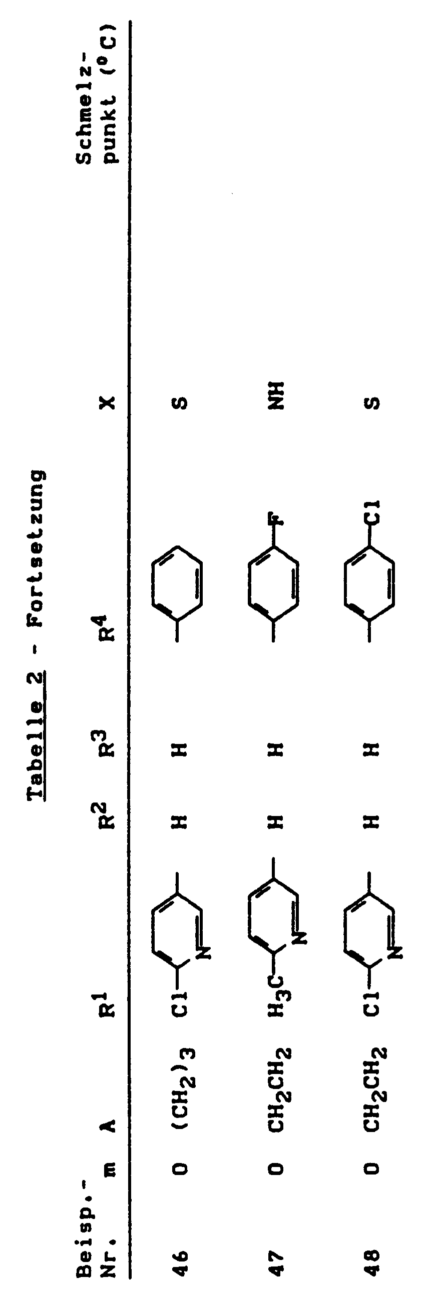

### Verwendungsbeispiele

In den nachfolgenden Verwendungsbeispielen wird die nachstehend angegebene Verbindung als Vergleichssubstanz eingesetzt: 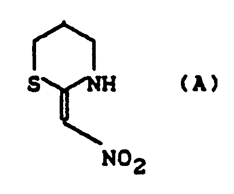
2-Nitromethylen-2H-tetrahydro-1,3-thiazin (vgl. US-PS 3 993 648).

### Beispiel A

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen die gemäß den Herstellungsbeispielen (1), (2), (3), (4), (5), (8), (11), (12), (15), (20) und (21) erhaltenen Verbindungen bei einer Wirkstoffkonzentration von 0,001 % eine Wirkung zwischen 50 % und 100 % nach 3 Tagen, während die Vergleichssubstanz (A) keine nachweisbare Wirkung zeigt.

### Beispiel B

### Spodoptera-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen die gemäß den Herstellungsbeispielen (1), (2), (4) und (11) erhaltenen Verbindungen bei einer Wirkstoffkonzentration von 0,001 % eine Wirkung zwischen 90 % und 100 % nach 3 Tagen, während die Vergleichssubstanz (A) keine nachweisbare Wirkung zeigt.

### Beispiel C

### Myzus-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen die gemäß den Herstellungsbeispielen (1), (3), (4), (5), (7), (15), (20) und (21) erhaltenen Verbindungen bei einer Wirkstoffkonzentration von 0,001 % eine Wirkung zwischen 80 % und 100 % nach einem Tag, währen die Vergleichssubstanz (A) nur eine Wirkung von 10 % zeigt.

### Beispiel D

### Grenzkonzentrations-Test / Wurzelsystemische Wirkung

- Testinsekt:: Phaedon cochleariae
- Lösungsmittel:: 3 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen die gemäß den Herstellungsbeispielen (1), (2), (3), (4) und (5) erhaltenen Verbindungen bei einer Wirkstoffkonzentration von 20 ppm eine Wirkung von 100 %, während die Vergleichssubstanz (A) keine nachweisbare Wirkung zeigt.

### Beispiel E

### Grenzkonzentrations-Test / Wurzelsystemische Wirkung

- Testinsekt:: Myzus persicae
- Lösungsmittel:: 3 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere aabgetötet sind und 0 %, wenn noch genau so viele Testinsekten leben wie bei der unbehandelten Kontrolle.

Bei diesem Test zeigen die gemäß den Herstellungsbeispielen (1), (2), (3), (4) und (5) erhaltenen Verbindungen bei einer Wirkstoffkonzentration von 20 ppm eine Wirkung von 100 %, während die Vergleichssubstanz (A) keine nachweisliche Wirkung zeigt.

### Beispiel F

### Test mit Lucilia cuprina resistent-Larven

- Emulgator:: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in eine Teströhrchen gebracht, welches ca. 1 cm³ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: (1), (2), (3), (4), (5), (7), (8) und (11).

## Patentansprüche

1. Substituierte Nitroalkene der allgemeinen Formel (I) 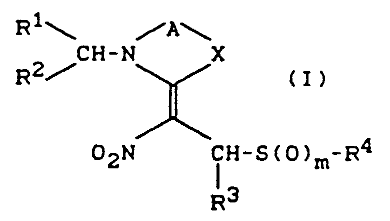 in welcher
m für die Zahlen 0, 1 oder 2 steht,
A für Alkandiyl steht,
R¹ für eine fünf- oder sechsgliedrige heterocyclische Gruppierung steht, welche 1, 2, 3 oder 4 Stickstoffatome und/oder ein oder zwei Sauerstoff- oder Schwefelatome als Heteroatom-Ringglieder enthält - wobei die Zahl der Heteroatome 1, 2, 3 oder 4 beträgt - und welche gegebenenfalls durch Halogen, Cyano, Nitro, Alkyl, Halogenalkyl, Alkenyl, Halogenalkenyl, Alkinyl, Alkoxy, Halogenalkoxy, Alkenyloxy, Halogenalkenyloxy, Alkinyloxy, Alkylthio, Halogenalkythio, Alkenylthio, Halogenalkenylthio, Alkinylthio, Alkylsulfinyl, Halogenalkylsulfinyl, Alkylsulfonyl, Halogenalkylsulfonyl, Amino, Alkylamino, Dialkylamino, Aryl, Aryloxy, Arylthio, Arylamino, Aralkyl, Formylamino, Alkylcarbonylamino, Formyl, Carbamoyl, Alkylcarbonyl und/oder Alkoxycarbonyl substituiert ist,
R² für Wasserstoff oder Alkyl steht,
R³ für Wasserstoff, Alkyl (welches gegebenenfalls durch Halogen, Cyano, Alkoxy oder Alkylthio substituiert ist), Alkenyl (welches gegebenenfalls durch Halogen oder Phenyl substituiert ist), Alkinyl, Phenyl (welches gegebenenfalls durch Halogen, Cyano, Nitro, Alkyl, Halogenalkyl, Alkoxy, Halogenalkoxy, Alkylthio, Halogenalkylthio, Alkylsulfinyl, Alkylsulfonyl, Halogenalkylsulfonyl, Dialkylamino oder Alkoxycarbonyl substituiert ist), Benzyl (welches gegebenenfalls durch Halogen, Cyano, Nitro, Alkyl, Halogenalkyl oder Alkoxycarbonyl substituiert ist), Furyl, Furylmethyl, Thenyl, Thienyl oder Pyridyl (welche gegebenenfalls durch Halogen oder Alkyl substituiert sind) steht,
R⁴ für Phenyl (welches gegebenenfalls durch Halogen, Cyano, Nitro, Alkyl, Halogenalkyl, Alkoxy, Halogenalkoxy, Alkylthio, Halogenalkylthio, Amino, Alkylamino, Dialkylamino oder Alkoxycarbonyl substituiert ist), Naphthyl (welches gegebenenfalls durch Halogen, Cyano, Nitro, Alkyl oder Amino substituiert ist), Pyridinyl (welches gegebenenfalls durch Halogen oder Alkyl substituiert ist), Pyrimidinyl (welches gegebenenfalls durch Halogen oder Alkyl substituiert ist), Imidazolyl oder Triazolyl steht und
X für Sauerstoff, Schwefel oder die Gruppierung N-R⁵ steht, worin
R⁵ für Wasserstoff, Alkyl (welches gegebenenfalls durch Halogen, Cyano, Alkoxy, Alkylthio, Dialkylamino, Trialkylsilyl, Alkoxycarbonyl, Carboxy, Carbamoyl, Alkylaminocarbonyl, Dialkylaminocarbonyl oder durch den Rest R¹ substituiert ist, wobei R¹ die oben angegebene Bedeutung besitzt), für Alkenyl (welches gegebenenfalls durch Halogen substituiert ist), Alkinyl, Benzyl (welches gegebenenfalls durch Halogen, Cyano, Nitro, Alkyl, Halogenalkyl, Alkoxy oder Alkoxycarbonyl substituiert ist), Formyl, Alkylcarbonyl (welches gegebenenfalls durch Halogen, Cyano, Phenyl, Phenoxy oder Alkoxy substituiert ist), Cycloalkylcarbonyl (welches gegebenenfalls durch Halogen und/oder Alkyl substituiert ist), Alkenylcarbonyl (welches gegebenenfalls durch Halogen substituiert ist), Phenylcarbonyl oder Naphthylcarbonyl (welche gegebenenfalls durch Halogen, Alkyl, Halogenalkyl, Cyano, Nitro, Alkoxy und/oder Alkoxycarbonyl substituiert sind), Alkoxycarbonyl, Benzyloxycarbonyl, Phenoxycarbonyl, Alkylthiocarbonyl, Benzylthiocarbonyl, Phenylthiocarbonyl, Alkylaminocarbonyl, Dialkylaminocarbonyl, Phenylaminocarbonyl (welches gegebenenfalls durch Halogen, Cyano, Nitro, Alkyl, Halogenalkyl, Alkoxy, Halogenalkoxy, Alkylthio, Halogenalkylthio oder Alkoxycarbonyl substituiert ist), Benzoylaminocarbonyl (welches gegebenenfalls durch Halogen, Alkyl oder Halogenalkyl substituiert ist), Phenylsulfonylaminocarbonyl (welches gegebenenfalls durch Halogen, Alkyl, Halogenalkyl, Alkoxy, Halogenalkoxy oder Alkoxycarbonyl substituiert ist), Alkylthio (welches gegebenenfalls durch Halogen substituiert ist), Phenylthio (welches gegebenenfalls durch Halogen, Nitro oder Alkyl substituiert ist), Alkylsulfinyl, Alkylsulfonyl (welches gegebenenfalls durch Halogen substituiert ist), Phenylsulfinyl (welches gegebenenfalls durch Halogen, Nitro oder Alkyl substituiert ist), Phenylsulfonyl oder Naphthylsulfonyl (welche gegebenenfalls durch Halogen, Cyano, Nitro, Alkyl, Halogenalkyl, Alkoxy, Halogenalkoxy und/oder Alkoxycarbonyl substituiert sind), Dialkyl(thio)phosphoryl, Alkyl-alkoxy(thio)phosphoryl oder Dialkoxy(thio)phosphoryl steht.

2. Substituierte Nitroalkene der allgemeinen Formel (I) gemäß Anspruch 1, in welcher
m für die Zahlen 0, 1 oder 2 steht,
A für C₂-C₅-Alkandiyl steht,
R¹ für eine fünf- bzw. sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4,-1,2,5- oder 1,3,4-Thiadiazolyl, Pyridyl, Pyridazinyl, Pyrimidinyl und Pyrazinyl steht, welche gegebenenfalls durch Fluor, Chlor, Brom, Iod, Cyano, Nitro, C₁-C₄-Alkyl (welches gegebenenfalls durch Fluor und/oder Chlor substituiert ist), C₂-C₄-Alkenyl (welches gegebenenfalls durch Fluor und/oder Chlor substituiert ist), C₂-C₄-Alkinyl, C₁-C₄-Alkoxy (welches gegebenenfalls durch Fluor und/oder Chlor substituiert ist), C₃-C₄-Alkenyloxy (welches gegebenenfalls durch Fluor und/oder Chlor substituiert ist), C₃-C₄-Alkinyloxy, C₁-C₄-Alkylthio (welches gegebenenfalls durch Fluor und/oder Chlor substituiert ist), C₃-C₄-Alkenylthio (welches gegebenenfalls durch Fluor und/oder Chlor substituiert ist), C₃-C₄-Alkinylthio, C₁-C₄-Alkylsulfinyl (welches gegebenenfalls durch Fluor und/oder Chlor substituiert ist), C₁-C₄-Alkylsulfonyl (welches gegebenenfalls durch Fluor und/oder Chlor substituiert ist), Amino, C₁-C₄-Alkylamino, Di-(C₁-C₄-alkyl)-amino, Phenyl, Phenoxy, Phenylthio, Phenylamino, Benzyl, Formylamino, C₁-C₄-Alkylcarbonylamino, Formyl, Carbamoyl, C₁-C₄-Alkylcarbonyl und/oder C₁-C₄-Alkoxy-carbonyl substituiert ist,
R² für Wasserstoff oder C₁-C₃-Alkyl steht,
R³ für Wasserstoff, C₁-C₄-Alkyl (welches gegebenenfalls durch Fluor, Chlor, Cyano, C₁-C₄-Alkoxy oder C₁-C₄-Alkylthio substituiert ist), C₂-C₄-Alkenyl (welches gegebenenfalls durch Fluor, Chlor oder Phenyl substituiert ist), C₂-C₄-Alkinyl, Phenyl (welches gegebenenfalls durch Fluor, Chlor, Brom, Cyano, Nitro, C₁-C₄-Alkyl, Trifluormethyl, C₁-C₄-Alkoxy, C₁-C₂-Fluoralkoxy, C₁-C₂-Chlorfluoralkoxy, C₁-C₄-Alkylthio, C₁-C₂-Fluoralkylthio, C₁-C₂-Chlorfluoralkylthio, C₁-C₄-Alkylsulfinyl, C₁-C₄-Alkylsulfonyl, C₁-C₂-Fluoralkylsulfonyl, C₁-C₂-Chlorfluoralkylsulfonyl, Di-(C₁-C₃-alkyl)-amino oder C₁-C₃-Alkoxy-carbonyl substituiert ist), Benzyl (welches gegebenenfalls durch Fluor, Chlor, Cyano, Nitro, C₁-C₂-Alkyl, Trifluormethyl oder C₁-C₃-Alkoxy-carbonyl substituiert ist), Furyl, Furylmethyl, Thenyl, Thienyl oder Pyridyl (welche gegebenenfalls durch Fluor, Chlor oder C₁-C₄-Alkyl substituiert sind) steht,
R⁴ für Phenyl (welches gegebenenfalls durch Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio (welches gegebenenfalls durch Fluor und/ oder Chlor substituiert ist), Amino, C₁-C₄-Alkylamino, Di-(C₁-C₄-alkyl)-amino oder C₁-C₄-Alkoxycarbonyl substituiert ist), Naphthyl (welches gegebenenfalls durch Chlor, Cyano, Nitro, Amino oder C₁-C₄-Alkyl substituiert ist), Pyridinyl (welches gegebenenfalls durch Fluor, Chlor, Brom oder C₁-C₄-Alkyl substituiert ist), Pyrimidinyl (welches gegebenenfalls durch Fluor, Chlor oder C₁-C₄-Alkyl substituiert ist), Imidazolyl oder Triazolyl steht und
X für Sauerstoff, Schwefel oder die Gruppierung N-R⁵ steht, worin
R⁵ für Wasserstoff, C₁-C₄-Alkyl (welches gegebenenfalls durch Fluor, Chlor, Cyano, C₁-C₄-Alkoxy, C₁-C₄-Alkylthio, Di-(C₁-C₄-alkyl)-amino, Trimethylsilyl, C₁-C₄-Alkoxy-carbonyl, Carboxy, Carbamoyl, C₁-C₄-Alkyl-aminocarbonyl, Di-(C₁-C₃-alkyl)-aminocarbonyl, oder durch den Rest R¹ substituiert ist, C₂-C₄-Alkenyl (welches gegebenenfalls durch Fluor oder Chlor substituiert ist), C₂-C₄-Alkinyl, Benzyl (welches gegebenenfalls durch Fluor, Chlor, Cyano, Nitro, C₁-C₂-Alkyl, Trifluormethyl, C₁-C₂-Alkoxy oder C₁-C₂-Alkoxy-carbonyl substituiert ist), Formyl, C₁-C₂₀-Alkyl-carbonyl (welches gegebenenfalls durch Fluor, Chlor, Brom, Cyano, Phenyl, Phenoxy oder C₁-C₄-Alkoxy substituiert ist), C₃-C₆-Cycloalkylcarbonyl (welches gegebenenfalls durch Fluor, Chlor und/oder C₁-C₄-Alkyl substituiert ist), C₂-C₂₀-Alkenyl-carbonyl (welches gegebenenfalls durch Fluor und/oder Chlor substituiert ist), Phenylcarbonyl oder Naphthylcarbonyl (welche gegebenenfalls durch Fluor, Chlor, Brom, C₁-C₄-Alkyl, Trifluormethyl, Cyano, Nitro, C₁-C₄-Alkoxy und/oder C₁-C₄-Alkoxy-carbonyl substituiert sind), C₁-C₂₀-Alkoxy-carbonyl, Benzyloxy-carbonyl, Phenoxy-carbonyl, C₁-C₄-Alkylthio-carbonyl, Benzylthio-carbonyl, Phenylthio-carbonyl, C₁-C₆-Alkylamino-carbonyl, Di-(C₁-C₄-alkyl)-amino-carbonyl, Phenylamino-carbonyl (welches gegebenenfalls durch Fluor, Chlor, Brom, Cyano, Nitro, C₁-C₄-Alkyl, Trifluormethyl, C₁-C₄-Alkoxy, C₁-C₂-Fluoralkoxy, C₁-C₂-Chlorfluoralkoxy, C₁-C₄-Alkylthio, C₁-C₂-Fluoralkylthio, C₁-C₂-Chlorfluoralkylthio oder C₁-C₄-Alkoxy-carbonyl substituiert ist), Benzoylamino-carbonyl (welches gegebenenfalls durch Fluor, Chlor, Brom, Methyl oder Trifluormethyl substituiert ist), Phenylsulfonylamino-carbonyl (welches gegebenenfalls durch Fluor, Chlor, Brom, Methyl, Trifluormethyl, C₁-C₄-Alkoxy, C₁-C₂-Fluoralkoxy, C₁-C₂-Chlorfluoralkoxy oder C₁-C₄-Alkoxy-carbonyl substituiert ist), C₁-C₄-Alkylthio (welches gegebenenfalls durch Fluor und/oder Chlor substituiert ist), Phenylthio (welches gegebenenfalls durch Fluor, Chlor, Brom, Nitro oder Methyl substituiert ist), C₁-C₄-Alkylsulfinyl, C₁-C₄-Alkylsulfonyl (welches gegebenenfalls durch Fluor und/oder Chlor substituiert ist), Phenylsulfinyl (welches gegebenenfalls durch Fluor, Chlor, Brom, Nitro oder Methyl substituiert ist) Phenylsulfonyl oder Naphthylsulfonyl (welche gegebenenfalls durch Fluor, Chlor, Brom, Cyano, Nitro, Methyl, Trifluormethyl, C₁-C₄-Alkoxy, C₁-C₂-Fluoralkoxy, C₁-C₂-Chlorfluoralkoxy und/oder C₁-C₄-Alkoxy-carbonyl substituiert sind), Dimethyl(thio)phosphoryl, C₁-C₄-Alkyl-C₁-C₄-alkoxy(thio)phosphoryl oder Di-(C₁-C₄-alkoxy)(thio)phosphoryl steht.

3. Substituierte Nitroalkene der allgemeinen Formel (I) gemäß Anspruch 1, in welcher
m für die Zahlen 0, 1 oder 2 steht,
A für Ethan-1,2-diyl (Dimethylen) oder Propan-1,3-diyl (Trimethylen) steht,
R¹ für eine fünf- bzw. sechsgliedrige heterocyclische Gruppierung aus der Reihe Pyrazolyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl steht, welche gegebenenfalls durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/ oder Chlor substituiert ist) substituiert ist,
R² für Wasserstoff steht,
R³ für Wasserstoff steht,
R⁴ für Phenyl (welches gegebenenfalls durch Fluor, Chlor, C₁-C₄-Alkyl, Trifluormethyl, C₁-C₂-Alkoxy (welches gegebenenfalls durch Fluor und/oder Chlor substituiert ist) oder C₁-C₂-Alkoxy-carbonyl substituiert ist) oder für Naphthyl steht und
X für Schwefel oder die Gruppierung N-R⁵ steht, worin
R⁵ für Wasserstoff, Methyl, Ethyl, Allyl, Propargyl, Formyl, C₁-C₈-Alkyl-carbonyl, Phenylcarbonyl (welches gegebenenfalls durch Fluor und/oder Chlor substituiert ist), C₁-C₈-Alkoxy-carbonyl, Benzyloxy-carbonyl, Phenoxy-carbonyl, Benzyl (welches gegebenenfalls durch Fluor oder Chlor substituiert ist) oder Di-(C₁-C₂-alkoxy)-(thio)phosphoryl steht.

4. Verfahren zur Herstellung von substituierten Nitroalkenen der allgemeinen Formel (I) 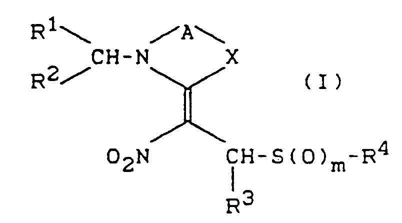 in welcher
m für die Zahlen 0, 1 oder 2 steht,
A für Alkandiyl steht,
R¹ für eine fünf- oder sechsgliedrige heterocyclische Gruppierung steht, welche 1, 2, 3 oder 4 Stickstoffatome und/oder ein oder zwei Sauerstoff- oder Schwefelatome als Heteroatom-Ringglieder enthält - wobei die Zahl der Heteroatome 1, 2, 3 oder 4 beträgt - und welche gegebenenfalls durch Halogen, Cyano, Nitro, Alkyl, Halogenalkyl, Alkenyl, Halogenalkenyl, Alkinyl, Alkoxy, Halogenalkoxy, Alkenyloxy, Halogenalkenyloxy, Alkinyloxy, Alkylthio, Halogenalkythio, Alkenylthio, Halogenalkenylthio, Alkinylthio, Alkylsulfinyl, Halogenalkylsulfinyl, Alkylsulfonyl, Halogenalkylsulfonyl, Amino, Alkylamino, Dialkylamino, Aryl, Aryloxy, Arylthio, Arylamino, Aralkyl, Formylamino, Alkylcarbonylamino, Formyl, Carbamoyl, Alkylcarbonyl und/oder Alkoxycarbonyl substituiert ist,
R² für Wasserstoff oder Alkyl steht,
R³ für Wasserstoff, Alkyl (welches gegebenenfalls durch Halogen, Cyano, Alkoxy oder Alkylthio substituiert ist), Alkenyl (welches gegebenenfalls durch Halogen oder Phenyl substituiert ist), Alkinyl, Phenyl (welches gegebenenfalls durch Halogen, Cyano, Nitro, Alkyl, Halogenalkyl, Alkoxy, Halogenalkoxy, Alkylthio, Halogenalkylthio, Alkylsulfinyl, Alkylsulfonyl, Halogenalkylsulfonyl, Dialkylamino oder Alkoxycarbonyl substituiert ist), Benzyl (welches gegebenenfalls durch Halogen, Cyano, Nitro, Alkyl, Halogenalkyl oder Alkoxycarbonyl substituiert ist), Furyl, Furylmethyl, Thenyl, Thienyl oder Pyridyl (welche gegebenenfalls durch Halogen oder Alkyl substituiert sind) steht,
R⁴ für Phenyl (welches gegebenenfalls durch Halogen, Cyano, Nitro, Alkyl, Halogenalkyl, Alkoxy, Halogenalkoxy, Alkylthio, Halogenalkylthio, Amino, Alkylamino, Dialkylamino oder Alkoxycarbonyl substituiert ist), Naphthyl (welches gegebenenfalls durch Halogen, Cyano, Nitro, Alkyl oder Amino substituiert ist), Pyridinyl (welches gegebenenfalls durch Halogen oder Alkyl substituiert ist), Pyrimidinyl (welches gegebenenfalls durch Halogen oder Alkyl substituiert ist), Imidazolyl oder Triazolyl steht und
X für Sauerstoff, Schwefel oder die Gruppierung N-R⁵ steht, worin
R⁵ für Wasserstoff, Alkyl (welches gegebenenfalls durch Halogen, Cyano, Alkoxy, Alkylthio, Dialkylamino, Trialkylsilyl, Alkoxycarbonyl, Carboxy, Carbamoyl, Alkylaminocarbonyl, Dialkylaminocarbonyl oder durch durch den Rest R¹ substituiert ist, wobei R¹ die oben angegebene Bedeutung besitzt), für Alkenyl (welches gegebenenfalls durch Halogen substituiert ist), Alkinyl, Benzyl (welches gegebenenfalls durch Halogen, Cyano, Nitro, Alkyl, Halogenalkyl, Alkoxy oder Alkoxycarbonyl substituiert ist), Formyl, Alkylcarbonyl (welches gegebenenfalls durch Halogen, Cyano, Phenyl, Phenoxy oder Alkoxy substituiert ist), Cycloalkylcarbonyl (welches gegebenenfalls durch Halogen und/oder Alkyl substituiert ist), Alkenylcarbonyl (welches gegebenenfalls durch Halogen substituiert ist), Phenylcarbonyl oder Naphthylcarbonyl (welche gegebenenfalls durch Halogen, Alkyl, Halogenalkyl, Cyano, Nitro, Alkoxy und/oder Alkoxycarbonyl substituiert sind), Alkoxycarbonyl, Benzyloxycarbonyl, Phenoxycarbonyl, Alkylthiocarbonyl, Benzylthiocarbonyl, Phenylthiocarbonyl, Alkylaminocarbonyl, Dialkylaminocarbonyl, Phenylaminocarbonyl (welches gegebenenfalls durch Halogen, Cyano, Nitro, Alkyl, Halogenalkyl, Alkoxy, Halogenalkoxy, Alkylthio, Halogenalkylthio oder Alkoxycarbonyl substituiert ist), Benzoylaminocarbonyl (welches gegebenenfalls durch Halogen, Alkyl oder Halogenalkyl substituiert ist), Phenylsulfonylaminocarbonyl (welches gegebenenfalls durch Halogen, Alkyl, Halogenalkyl, Alkoxy, Halogenalkoxy oder Alkoxycarbonyl substituiert ist), Alkylthio (welches gegebenenfalls durch Halogen substituiert ist), Phenylthio (welches gegebenenfalls durch Halogen, Nitro oder Alkyl substituiert ist), Alkylsulfinyl, Alkylsulfonyl (welches gegebenenfalls durch Halogen substituiert ist), Phenylsulfinyl (welches gegebenenfalls durch Halogen, Nitro oder Alkyl substituiert ist), Phenylsulfonyl oder Naphthylsulfonyl (welche gegebenenfalls durch Halogen, Cyano, Nitro, Alkyl, Halogenalkyl, Alkoxy, Halogenalkoxy und/oder Alkoxycarbonyl substituiert sind), Dialkyl(thio)phosphoryl, Alkylalkoxy-(thio)phosphoryl oder Dialkoxy(thio)phosphoryl steht,
dadurch gekennzeichnet, daß man
a) zum Erhalt von Verbindungen der Formel (I), in welcher A, R¹, R², R³, R⁴, X und m die oben angegebene Bedeutung besitzen,
Nitroalkene der allgemeinen Formel (II) 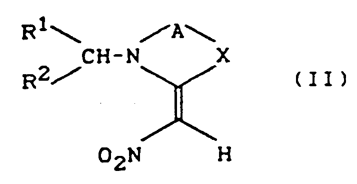 in welcher
A, R¹, R² und X die oben angegebenen Bedeutungen haben,
- bzw. die Isomeren der korrespondierenden Konfiguration (Z bzw. E) bzw. Gemische der Z- und E-Isomeren -
mit Aldehyden der allgemeinen Formel (III) 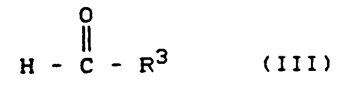 in welcher
R³ die oben angegebenen Bedeutung hat,
und mit Mercaptoverbindungen der allgemeinen Formel (IV)
HS - R⁴ (IV)
in welcher
R⁴ die oben angegebene Bedeutung hat,
gegebenenfalls in Gegenwart einer Säure und gegebenenfalls in Gegenwart eines Verdünnungsmittels umsetzt und gegebenenfalls die so erhaltenen Verbindungen der Formel (I), in welcher m für die Zahl 0 steht und A, R¹, R², R³, R⁴ und X die oben angegebenen Bedeutungen haben, nach üblichen Oxidationsmethoden in entsprechende Verbindungen der Formel (I), in welcher dann m für die Zahlen 1 oder 2 steht und A, R¹, R², R³, R⁴ und X die oben angegebenen Bedeutungen haben, umwandelt,
oder daß man
b) zum Erhalt von Verbindungen der Formel (I), in welcher A, R¹, R², R³, R⁴ und m die oben angegebene Bedeutung haben und X für die Gruppierung NR⁵ steht, wobei R⁵ die unter Formel (I) angegebene Bedeutung besitzt, Nitroalkene der Formel (Ia) 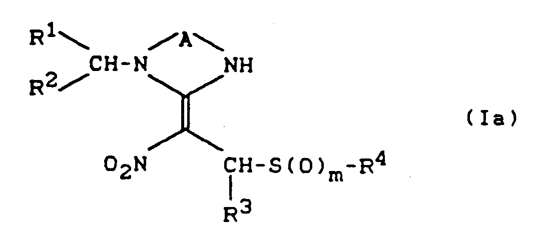 mit Halogenverbindungen der Formel (VII)
Z-R⁵ (VII)
in welcher
R⁵ die oben angegebene Bedeutung hat und
Z für Halogen steht,
gegebenenfalls in Gegenwart eines Säureakzeptors und gegebenenfalls in Gegenwart eines Verdünnungsmittels bei Temperaturen zwischen 10°C und 100°C umsetzt,
oder daß man
c) zum Erhalt von Verbindungen der Formel (I), in welcher A, R¹, R², R³, R⁴ und m die oben angegebene Bedeutung besitzen, Amine der Formel (VIII) 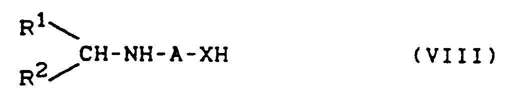 in welcher
R¹, R², A und X die oben angegebene Bedeutung besitzen, mit
1,1-Bismethylthio-3-nitro-alkenen der Formel (IX) 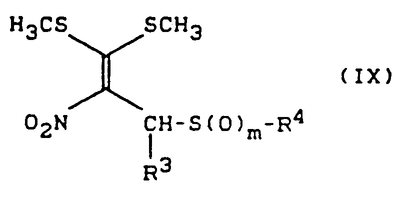 in welcher
R³, R⁴ und m die oben angegebene Bedeutung haben,
gegebenenfalls in Gegenwart eines Verdünnungsmittels bei Temperaturen zwischen 10°C und 150°C umsetzt, oder daß man
d) Halogenverbindungen der Formel V 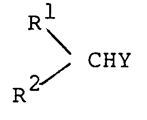 in welcher
R¹, R² die oben angegebene Bedeutung haben
Y für Halogen steht,
mit Nitroalkenen der Formel VI 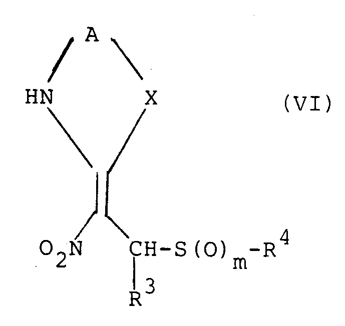 in welcher
A, R³, R⁴, X, m die oben angegebene Bedeutung besitzen,
gegebenenfalls in Gegenwart eines Säureakzeptors und gegebenenfalls in Gegenwart eines Verdünnungsmittels bei Temperaturen zwischen 10 und 100°C umsetzt.

5. Schädlingsbekämpfungsmittel, gekennzeichnet durch einen Gehalt an mindestens einem substituierten Nitroalken der Formel (I).

6. Insektizide Mittel, gemäß Anspruch 5 gekennzeichnet durch einen Gehalt an mindestens einem substituierten Nitroalken der Formel (I).

7. Verfahren zur Bekämpfung von Insekten, dadurch gekennzeichnet, daß man substituierte Nitroalkene der Formel (I) auf Insekten und/oder deren Lebensraum einwirken läßt.

8. Verwendung von substituierten Nitroalkenen der Formel (I) zur Bekämpfung von Insekten.

9. Verfahren zur Herstellung von Schädlingsbekämpfungsmitteln, dadurch gekennzeichnet, daß man substituierte Nitroalkene der Formel (I) mit Streckmitteln und/oder oberflächenaktiven Mitteln vermischt.

## Claims

1. Substituted nitroalkenes of the general formula (I) 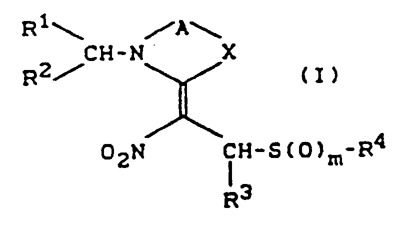 in which
m represents the numbers 0, 1 or 2
A represents alkanediyl,
R¹ represents a five-or six-membered heterocyclic grouping which contains 1, 2, 3 or 4 nitrogen atoms and/or one or two oxygen or sulphur atoms as heteroatom ring members - the number of heteroatoms being 1, 2, 3 or 4 - and which is optionally substituted by halogen, cyano, nitro, alkyl, halogenoalkyl, alkenyl, halogenoalkenyl, alkinyl, alkoxy, halogenoalkoxy, alkenyloxy, halgenoalkenyloxy, alkinyloxy, alkylthio, halogenoalkylthio, alkenylthio, halogenoalkenylthio, alkinylthio, alkylsulphinyl, halogenoalkylsulphinyl, alkylsulphonyl, halogenoalkylsulphonyl, amino, alkylamino, dialkylamino, aryl, aryloxy, arylthio, arylamino, aralkyl, formylamino, alkylcarbonyl-amino, formyl, carbamoyl, alkylcarbonyl and/or alkoxycarbonyl,
R² represents hydrogen or alkyl,
R³ represents hydrogen, alkyl (which is optionally substituted by halogen, cyano, alkoxy or alkylthio), alkenyl (which is optionally substituted by halogen or phenyl), alkinyl, phenyl (which is optionally substituted by halogen, cyano, nitro, alkyl, halogenoalkyl, alkoxy, halogenoalkoxy, alkylthio, halogenoalkylthio, alkylsulphinyl, alkylsulphonyl, halogenoalkylsulphonyl, dialkylamino or alkoxycarbonyl), benzyl (which is optionally substituted by halogen, cyano, nitro, alkyl, halogenoalkyl or alkoxycarbonyl), furyl, furylmethyl, thenyl, thienyl or pyridyl (which are optionally substituted by halogen or alkyl),
R⁴ represents phenyl (which is optionally substituted by halogen, cyano, nitro, alkyl, halogenoalkyl, alkoxy, halogenoalkoxy, alkylthio, halogenoalkylthio, amino, alkylamino, dialkylamino or alkoxycarbonyl), naphthyl (which is optionally substituted by halogen, cyano, nitro, alkyl or amino), pyridinyl (which is optionally substituted by halogen or alkyl), pyrimidinyl (which is optionally substituted by halogen or alkyl), imidazolyl or triazolyl and
X represents oxygen, sulphur or the grouping N-R⁵, wherein
R⁵ represents hydrogen, alkyl (which is optionally substituted by halogen, cyano, alkoxy, alkylthio, dialkylamino, trialkylsilyl, alkoxycarbonyl, carboxyl, carbamoyl, alkylaminocarbonyl, dialkylaminocarbonyl or by the radical R¹, R¹ possessing the abovementioned meaning), alkenyl ((which is optionally substituted by halogen), alkinyl, benzyl which is optionally substituted by halogen, cyano, nitro, alkyl, halogenoalkyl, alkoxy or alkoxycarbonyl), formyl, alkylcarbonyl (which is optionally substituted by halogen, cyano, phenyl, phenoxy or alkoxy), cycloalkylcarbonyl (which is optionally substituted by halogen and/or alkyl), alkenylcarbonyl, (which is optionally substituted by halogen), phenylcarbonyl or naphthylcarbonyl (which are optionally substituted by halogen, alkyl, halogenoalkyl, cyano, nitro, alkoxy and/or alkoxycarbonyl), alkoxycarbonyl, benzyloxycarbonyl, phenoxycarbonyl, alkylthiocarbonyl, benzylthiocarbonyl, phenylthiocarbonyl, alkylaminocarbonyl, dialkylaminocarbonyl, phenylaminocarbonyl (which is optionally substituted by halogen, cyano, nitro, alkyl, halogenoalkyl, alkoxy, halogenoalkoxy, alkylthio, halogenoalkylthio or alkoxycarbonyl), benzoylaminocarbonyl (which is optionally substituted by halogen, alkyl or halogenoalkyl), phenylsulphonylaminocarbonyl (which is optionally substituted by halogen, alkyl, halogenoalkyl, alkoxy, halogenoalkoxy or alkoxycarbonyl), alkylthio (which is optionally substituted by halogen), phenylthio (which is optionally substituted by halogen, nitro or alkyl), alkylsulphinyl, alkylsulphonyl (which is optionally substituted by halogen), phenylsulphinyl (which are optionally substituted by halogen, nitro or alkyl), phenylsulphonyl or naphthylsulphonyl (which is optionally substituted by halogen, cyano, nitro, alkyl, halogenoalkyl, alkoxy, halogenoalkoxy and/or alkoxycarbonyl), dialkyl(thio)phosphoryl, alkylalkoxy(thio)phosphoryl or dialkoxy(thio)phosphoryl.

2. Substituted nitroalkenes of the general formula (I) according to Claim 1 in which
m represents the numbers 0, 1 or 2,
A represents C₂-C₅-alkanediyl,
R¹ represents a five-or six-membered heterocyclic grouping from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl, and pyrazinyl, each of which is optionally substituted by fluorine, chlorine, bromine, iodine, cyano, nitro, C₁-C₄-alkyl (which is optionally substituted by fluorine and/or chlorine), C₂-C₄-alkenyl (which is optionally substituted by fluorine and/or chlorine), C₂-C₄-alkinyl, C₁-C₄-alkoxy (which is optionally substituted by fluorine and/or chlorine), C₃-C₄-alkenyloxy (which is optionally substituted by fluorine and/or chlorine), C₃-C₄-alkinyloxy, C₁-C₄-alkylthio (which is optionally substituted by fluorine and/or chlorine), C₃-C₄-alkenylthio (which is optionally substituted by fluorine and/or chlorine), C₃-C₄-alkinylthio, C₁-C₄-alkylsulphinyl (which is optionally substituted by fluorine and/or chlorine), C₁-C₄-alkylsulphonyl (which is optionally substituted by fluorine and/or chlorine), amino, C₁-C₄-alkyl-amino, di-(C₁-C₄-alkyl)amino, phenyl, phenoxy, phenylthio, phenylamino, benzyl, formylamino, C₁-C₄-alkylcarbonylamino, formyl, carbamoyl, C₁-C₄-alkylcarbonyl and/or C₁-C₄-alkoxy-carbonyl,
R² represents hydrogen or C₁-C₃-alkyl,
R³ represents hydrogen, C₁-C₄-alkyl (which is optionally substituted by fluorine, chlorine, cyano, C₁-C₄-alkoxy or C₁-C₄-alkylthio), C₂-C₄-alkenyl (which is optionally substituted by fluorine, chlorine or phenyl), C₂-C₄-alkinyl, phenyl (which is optionally substituted by fluorine, chlorine, bromine, cyano, nitro, C₁-C₄-alkyl, trifluoromethyl, C₁-C₄-alkoxy, C₁-C₂-fluoroalkoxy, C₁-C₂-chlorofluoroalkoxy, C₁-C₄-alkylthio C₁-C₂-fluoroalkylthio, C₁-C₂-chlorofluoroalkylthio, C₁-C₄-alkylsulphinyl, C₁-C₄-alkylsulphonyl, C₁-C₂-fluoroalkylsulphonyl, C₁-C₂-chlorofluoroalkylsulphonyl, di(C₁-C₃-alkyl)-amino or C₁-C₃-alkoxy-carbonyl), benzyl (which is optionally substituted by fluorine, chlorine, cyano, nitro, C₁-C₂-alkyl, trifluoromethyl or C₁-C₃-alkoxycarbonyl), furyl, furylmethyl, thenyl, thienyl or pyridyl (which are optionally substituted by fluorine, chlorine or C₁-C₄-alkyl),
R⁴ represents phenyl (which is optionally substituted by fluorine, chlorine, bromine, cyano, nitro, C₁-C₄-alkyl (which is optionally substituted by fluorine and/or chlorine), C₁-C₄-alkoxy, (which is optionally substituted by fluorine and/or chlorine), C₁-C₄-alkylthio (which is optionally substituted by fluorine and/or chlorine), amino C₁-C₄-alkylamino, di-(C₁-C₄-alkyl)-amino or C₁-C₄-alkoxycarbonyl), naphthyl (which is optionally) substituted by chlorine, cyano, nitro, amino or C₁-C₄-alkyl), pyridinyl (which is optionally substituted by fluorine, chlorine, bromine or C₁-C₄-alkyl), pyrimidinyl (which is optionally substituted by fluorine, chlorine or C₁-C₄-alkyl), imidazolyl or triazolyl and
X represents oxygen, sulphur or the grouping N-R⁵, wherein
R⁵ represents hydrogen, C₁-C₄-alkyl (which is optionally substituted by fluorine, chlorine, cyano, C₁-C₄-alkoxy, C₁-C₄-alkylthio, di-(C₁-C₄-alkyl)-amino, trimethylsilyl, C₁-C₄-alkoxycarbonyl, carboxyl, carbamoyl, C₁-C₄-alkyl-aminocarbonyl, di(C₁-C₃-alkyl)-aminocarbonyl, or by the radical R¹), C₂-C₄-alkenyl (which is optionally substituted by fluorine or chlorine), C₂-C₄-alkinyl, benzyl (which is optionally substituted by fluorine, chlorine, cyano, nitro, C₁-C₂-alkyl, trifluoromethyl, C₁-C₂-alkoxy or C₁-C₂-alkoxycarbonyl, formyl, C₁-C₂₀-alkylcarbonyl (which is optionally substituted by fluorine, chlorine, bromine, cyano, phenyl, phenoxy or C₁-C₄-alkoxy), C₃-C₆-cycloalkyl-carbonyl (which are optionally substituted by fluorine, chlorine and/or C₁-C₄-alkyl), C₂-C₂₀-alkenyl-carbonyl (which is optionally substituted by fluorine and/or chlorine) phenylcarbonyl or naphthylcarbonyl (which are optionally substituted by fluorine, chlorine, bromine, C₁-C₄-alkyl, trifluoromethyl, cyano, nitro, C₁-C₄-alkoxy and/or C₁-C₄-alkoxycarbonyl), C₁-C₂₀-alkoxycarbonyl, benzyloxycarbonyl, phenoxycarbonyl, C₁-C₄-alkylthio-carbonyl, benzylthio-carbonyl, phenylthio-carbonyl, C₁-C₆-alkylamino-carbonyl, di-(C₁-C₄-alkyl)-amino-carbonyl, phenylamino-carbonyl (which is optionally substituted by fluorine, chlorine, bromine, cyano, nitro, C₁-C₄-alkyl, trifluoromethyl, C₁-C₄-alkoxy, C₁-C₂-fluoroalkoxy, C₁-C₂-chlorofluoroalkoxy, C₁-C₄-alkylthio, C₁-C₂-fluoroalkylthio, C₁-C₂-chlorofluoroalkylthio or C₁-C₄-alkoxycarbonyl), benzoyl-amino-carbonyl (which is optionally substituted by fluorine, chlorine, bromine, methyl or trifluoromethyl), phenylsulphonylaminocarbonyl (which is optionally substituted by fluorine, chlorine, bromine, methyl, trifluoromethyl, C₁-C₄-alkoxy, C₁-C₂-fluoroalkoxy, C₁-C₂-chlorofluoroalkoxy or C₁-C₄-alkoxy-carbonyl), C₁-C₄-alkylthio (which is optionally substituted by fluorine and/or chlorine), phenylthio (which is optionally substituted by fluorine, chlorine, bromine, nitro or methyl), C₁-C₄-alkylsulphinyl, C₁-C₄-alkylsulphonyl (which is optionally substituted by fluorine and/or chlorine), phenylsulphinyl (which is optionally substituted by fluorine, chlorine, bromine, nitro or methyl), phenylsulphonyl or naphthylsulphonyl (which are optionally substituted by fluorine, chlorine, bromine, cyano, nitro, methyl, trifluoromethyl, C₁-C₄-alkoxy, C₁-C₂-fluoroalkoxy, C₁-C₂-chlorofluoroalkoxy and/or C₁-C₄-alkoxy-carbonyl), dimethyl(thio)phosphoryl, C₁-C₄-alkyl-C₁-C₄-alkoxy-(thio)-phosphoryl or di-(C₁-C₄-alkoxy)-(thio)phosphoryl.

3. Substituted nitroalkenes of the general formula (I) according to Claim 1, in which
m represents the numbers 0, 1 or 2,
A represents ethane-1,2-diyl (dimethylene) or propane-1,3-diyl (trimethylene),
R¹ represents a five- or six-membered heterocyclic grouping from the series comprising pyrazolyl, 1,2,4-triazolyl, oxazolyl, isoxazolyl, thiazolyl, isothiazolyl, 1,2,5-thiadiazolyl, pyridyl, pyrazinyl and pyrimidinyl, each of which is optionally substituted by fluorine, chlorine, bromine, cyano, nitro, C₁-C₂-alkyl (which is optionally substituted by fluorine and/or chlorine), C₁-C₂-alkoxy (which is optionally substituted by fluorine and/or chlorine), C₁-C₂-alkylthio (which is optionally substituted by fluorine and/or chlorine)- or C₁-C₂-alkylsulphonyl (which is optionally substituted by fluorine and/or chlorine),
R² represents hydrogen,
R³ represents hydrogen,
R⁴ represents phenyl (which is optionally substituted by fluorine, chlorine, C₁-C₄-alkyl, trifluoromethyl, C₁-C₂-alkoxy (which is optionally substituted by fluorine and/or chlorine) or C₁-C₂-alkoxy-carbonyl) or naphthyl and
X represents sulphur or the grouping N-R⁵, wherein,
R⁵ represents hydrogen, methyl, ethyl, allyl, propargyl, formyl, C₁-C₈-alkylcarbonyl, phenyl-carbonyl (which is optionally substituted by fluorine and/or chlorine), C₁-C₈-alkoxy-carbonyl, benzyloxycarbonyl, phenoxy-carbonyl, benzyl (which is optionally substituted by fluorine or chlorine) or di-(C₁-C₂-alkoxy)-(thio)phosphoryl.

4. Process for the preparation of substituted nitroalkenes of the general formula (I) 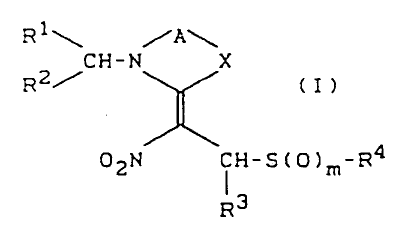 in which
m represents the numbers 0, 1 or 2,
A represents alkanediyl,
R¹ represents a five- or six-membered heterocyclic grouping which contains 1, 2, 3 or 4 nitrogen atoms and/or one or two oxygen or sulphur atoms as heteroatom ring members - the number of heteroatoms being 1, 2, 3 or 4- and which is optionally substituted by halogen, cyano, nitro, alkyl, halogenoalkyl, alkenyl, halogenoalkenyl, alkinyl, alkoxy, halogenoalkoxy, alkenyloxy, halogenoalkenyloxy, alkinyloxy, alkylthio, halogenoalkylthio, alkenylthio, halogenoalkenylthio, alkinylthio, alkylsulphinyl, halogenoalkylsulphinyl, alkylsulphonyl, halogenoalkylsulphonyl, amino, alkylamino, dialkylamino, aryl, aryloxy, arylthio, arylamino, aralkyl, formylamino, alkylcarbonylamino, formyl, carbamoyl, alkylcarbonyl and/or alkoxycarbonyl,
R² represents hydrogen or alkyl
R³ represents hydrogen, alkyl (which is optionally substituted by halogen, cyano, alkoxy or alkylthio), alkenyl (which is optionally substituted by halogen or phenyl), alkinyl, phenyl (which is optionally substituted by halogen, cyano, nitro, alkyl, halogenoalkyl, alkoxy, halogenoalkoxy, alkylthio, halogenoalkylthio, alkylsulphinyl, alkylsulphonyl, halogenoalkylsulphonyl, dialkylamino or alkoxycarbonyl), benzyl (which is optionally substituted by halogen, cyano, nitro, alkyl, halogenoalkyl or alkoxycarbonyl), furyl, furyl-methyl, thenyl, thienyl or pyridyl (which is optionally substituted by halogen or alkyl),
R⁴ represents phenyl (which is optionally substituted by halogen, cyano, nitro, alkyl, halogenoalkyl, alkoxy, halogenoalkoxy, alkylthio, halogenoalkylthio, amino, alkylamino, dialkylamino or alkoxycarbonyl), naphthyl (which is optionally substituted by halogen, cyano, nitro, alkyl or amino), pyridinyl (which is optionally substituted by halogen or alkyl), pyrimidinyl (which is optionally substituted by halogen or alkyl), imidazolyl or triazolyl and
X represents oxygen, sulphur or the grouping N-R⁵, wherein
R⁵ represents hydrogen, alkyl (which is optionally substituted by halogen, cyano, alkoxy, alkylthio, dialkylamino, trialkylsilyl, alkoxycarbonyl, carboxyl, carbamoyl, alkylaminocarbonyl, dialkylaminocarbonyl or by the radical R¹, R¹ possessing the abovementioned meaning), alkenyl (which is optionally substituted by halogen), alkinyl, benzyl (which is optionally substituted by halogen, cyano, nitro, alkyl, halogenoalkyl, alkoxy or alkoxycarbonyl), formyl, alkylcarbonyl (which is optionally substituted by halogen, cyano, phenyl, phenoxy or alkoxy), cycloalkylcarbonyl (which is optionally substituted by halogen and/or alkyl), alkenylcarbonyl (which is optionally substituted by halogen), phenylcarbonyl or naphthylcarbonyl (which are optionally substituted by halogen, alkyl, halogenoalkyl, cyano, nitro, alkoxy and/or alkoxycarbonyl), alkoxycarbonyl, benzyloxycarbonyl, phenoxycarbonyl, alkylthiocarbonyl, benzylthiocarbonyl, phenylthiocarbonyl, alkylaminocarbonyl, dialkylaminocarbonyl, phenylaminocarbonyl (which is optionally substituted by halogen, cyano, nitro, alkyl, halogenoalkyl, alkoxy, halogenoalkoxy, alkylthio, halogenoalkylthio or alkoxycarbonyl), benzoylaminocarbonyl (which is optionally substituted by halogen, alkyl or halogenoalkyl), phenylsulphonylaminocarbonyl (which is optionally substituted by halogen, alkyl, halogenoalkyl, alkoxy, halogenoalkoxy or alkoxycarbonyl),alkylthio (which is optionally substituted by halogen), phenylthio (which is optionally substituted by halogen, nitro or alkyl), alkylsulphinyl, alkylsulphonyl (which is optionally substituted by halogen), phenylsulphinyl (which is optionally substituted by halogen, nitro or alkyl), phenylsulphonyl or naphthylsulphonyl (which are optionally substituted by halogen, cyano, nitro, alkyl, halogenoalkyl, alkoxy, halogenoalkoxy and/or alkoxycarbonyl), dialkyl(thio)phosphoryl, alkylalkoxy-(thio)phosphoryl or dialkoxy(thio)phosphoryl,
characterised in that
(a) to obtain compounds of the formula (I), in which A, R¹, R², R³, R⁴, X and m possess the abovementioned meaning,
nitroalkenes of the general formula (II) 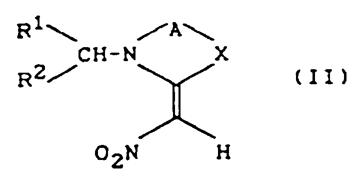 in which
A, R¹, R² and X have the abovementioned meanings,
- or the isomers of the corresponding configuration (Z or E) or mixtures of the Z and E isomers -
are reacted with aldehydes of the general formula (III) 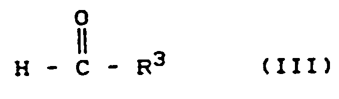 in which
R³ has the abovementioned meaning,
and with mercapto compounds of the general formula (IV)
HS - R⁴ (IV)
in which
R⁴ has the abovementioned meaning,
if appropriate in the presence of an acid and if appropriate in the presence of a diluent, and the resultant compounds of the formula (I), in which m represents the number 0 and A, R¹, R², R³, R⁴ and X have the abovementioned meanings, are converted, if desired, by customary oxidation methods into corresponding compounds of the formula (I) in which m then represents the numbers 1 or 2 and A, R¹, R², R³, R⁴ and X have the abovementioned meanings,
or in that
b) to obtain compounds of the formula (I), in which A, R¹, R², R³, R⁴ and m have the abovementioned meaning and X represents the grouping NR⁵, in which R⁵ possesses the meaning given under formula (I), nitroalkenes of the formula (Ia) 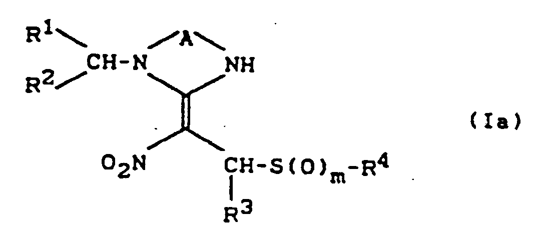 are reacted with halogen compounds of the formula (VII)
Z-R⁵ (VII)
in which
R⁵ has the abovementioned meaning and
Z represents halogen,
if appropriate in the presence of an acid acceptor and if appropriate in the presence of a diluent at temperatures between 10°C and 100°C,
or in that
c) to obtain compounds of the formula (I), in which A, R¹, R², R³, R⁴ and m possess the abovementioned meaning, amines of the formula (VIII) 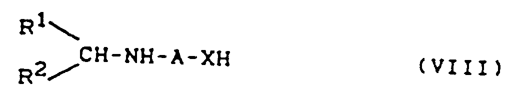 in which
R¹, R², A and X possess the abovementioned meaning,
are reacted with 1,1-bismethylthio-3-nitroalkenes of the formula (IX) 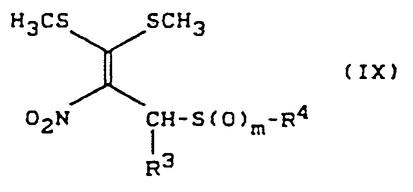 in which
R³, R⁴ and m have the abovementioned meaning,
if appropriate in the presence of a diluent at temperatures between 10°C and 150°C, or in that
d) halogen compounds of the formula V 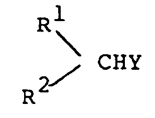 in which
R¹, R² have the abovementioned meaning and
Y represents halogen
are reacted with nitroalkenes of the formula VI 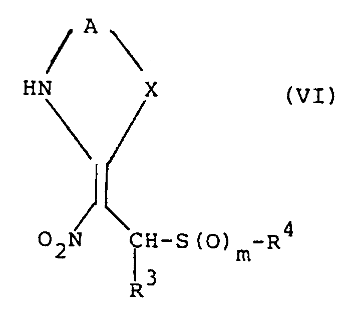 in which
A, R³, R⁴, X, m have the abovementioned meaning
if appropriate in the presence of an acid acceptor and if appropriate in the presence of a diluent at temperatures between 10 and 100°C.

5. Pesticides, characterized in that they contain at least one substituted nitroalkene of the formula (I).

6. Insecticidal agents according to Claim 5, characterised in that they contain at least one substituted nitroalkene of the formula (I).

7. Method of combating insects, characterized in that substituted nitroalkenes of the formula (I) are allowed to act on insects and/or their environment.

8. Use of substituted nitroalkenes of the formula (I) for combating insects.

9. Process for the production of pesticides, characterized in that substituted nitroalkenes of the formula (I) are mixed with extenders and/or surface-active agents.

## Revendications

1. Nitroalcènes substitués de formule générale (I) 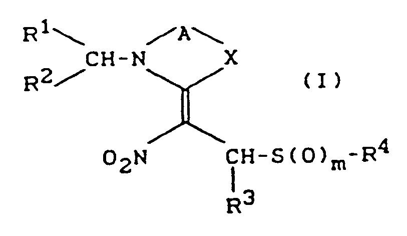 dans laquelle
m représente le nombre 0, 1 ou 2,
A est un groupe alcanediyle,
R¹ est un groupement hétérocyclique pentagonal ou hexagonal qui comporte 1, 2, 3 ou 4 atomes d'azote et/ou un ou deux atomes d'oxygène ou de soufre comme termes hétéro-atomiques du noyau - le nombre d'hétéro-atomes s'élevant à 1, 2, 3 ou 4 - et qui est substitué le cas échéant par un halogène, un groupe cyano, nitro, alkyle, halogénalkyle, alcényle, halogénalcényle, alcynyle, alkoxy, halogénalkoxy, alcényloxy, halogénalcényloxy, alcynyloxy, alkylthio, halogénalkylthio, alcénylthio, halogénalcénylthio, alcynylthio, alkylsulfinyle, halogénalkylsulfinyle, alkylsulfonyle, halogénalkylsulfonyle, amino, alkylamino, dialkylamino, aryle, aryloxy, arylthio, arylamino, aralkyle, formylamino, alkylcarbonylamino, formyle, carbamoyle, alkylcarbonyle et/ou alkoxycarbonyle,
R² est l'hydrogène ou un groupe alkyle,
R³ est l'hydrogène, un groupe alkyle (qui est substitué le cas échéant par un halogène, un radical cyano, alkoxy ou alkylthio), un groupe alcényle (qui est substitué le cas échéant par un halogène ou un radical phényle), un groupe alcynyle, phényle (qui est substitué le cas échéant par un halogène, un radical cyano, nitro, alkyle, halogénalkyle, alkoxy, halogénalkoxy, alkylthio, halogénalkylthio, alkylsulfinyle, alkylsulfonyle, halogénalkylsulfonyle, dialkylamino ou alkoxycarbonyle), un groupe benzyle (qui est substitué le cas échéant par un halogène, un radical cyano, nitro, alkyle, halogénalkyle ou alkoxycarbonyle), un groupe furyle, furylméthyle, thényle, thiényle ou pyridyle (chacun étant substitué le cas échéant par un halogène ou un radical alkyle),
R⁴ est un groupe phényle (qui est substitué le cas échéant par un halogène, un radical cyano, nitro, alkyle, halogénalkyle, alkoxy, halogénalkoxy, alkylthio, halogénalkylthio, amino, alkylamino, dialkylamino ou alkoxycarbonyle), un groupe naphtyle (qui est substitué le cas échéant par un halogène, un radical cyano, nitro, alkyle ou amino), un groupe pyridinyle (qui est substitué le cas échéant par un halogène ou un radical alkyle), un groupe pyrimidinyle (qui est substitué le cas échéant par un halogène ou un radical alkyle), un groupe imidazolyle ou triazolyle et
X représente l'oxygène, le soufre ou le groupement N-R⁵, dans lequel
R⁵ représente l'hydrogène, un groupe alkyle (qui est substitué le cas échéant par un halogène, un radical cyano, alkoxy, alkylthio, dialkylamino, trialkylsilyle, alkoxycarbonyle, carboxy, carbamoyle, alkylaminocarbonyle, dialkylaminocarbonyle ou par le reste R¹, lequel a la définition indiquée ci-dessus), un groupe alcynyle (qui est substitué le cas échéant par un halogène, un groupe alcinyle, benzyle (qui est substitué le cas échéant par un halogène, un radical cyano, nitro, alkyle, halogénalkyle, alkoxy ou alkoxycarbonyle), un groupe formyle, alkylcarbonyle (qui est substitué le cas échéant par un halogène, un radical cyano, phényle, phénoxy ou alkoxy), un groupe cycloalkylcarbonyle (qui est substitué le cas échéant par un halogène et/ou un radical alkyle), un groupe alcénylcarbonyle (qui est substitué le cas échéant par un halogène), un groupe phénylcarbonyle ou naphtylcarbonyle (qui est substitué le cas échéant par un halogène, un radical alkyle, halogénalkyle, cyano, nitro, alkoxy et/ou alkoxycarbonyle), un groupe alkoxycarbonyle, benzyloxycarbonyle, phénoxycarbonyle, alkylthiocarbonyle, benzylthiocarbonyle,phénylthiocarbonyle, alkylaminocarbonyle, dialkylaminocarbonyle, phénylaminocarbonyle (qui est substitué le cas échéant par un halogène, un radical cyano, nitro, alkyle, halogénalkyle, alkoxy, halogénalkoxy, alkylthio, halogénalkylthio ou alkoxycarbonyle), un groupe benzoylaminocarbonyle (qui est substitué le cas échéant par un halogène, un radical alkyle ou halogénalkyle), un groupe phénylsulfonylaminocarbonyle (qui est substitué le cas échéant par un halogène, un radical alkyle, halogénalkyle, alkoxy, halogénalkoxy ou alkoxycarbonyle), un groupe alkylthio (qui est substitué le cas échéant par un halogène), un groupe phénylthio (qui est substitué le cas échéant par un halogène, un radical nitro ou alkyle), un groupe alkylsulfinyle, alkylsulfonyle (qui est substitué le cas échéant par un halogène, un groupe phénylsulfinyle (qui est substitué le cas échéant par un halogène, un radical nitro ou alkyle), un groupe phénylsulfonyle ou un groupe naphtylsulfonyle (qui sont substitués le cas échéant par un halogène, un radical cyano, nitro, alkyle, halogénalkyle, alkoxy, halogénalkoxy et/ou alkoxycarbonyle), un groupe dialkyl(thio)phosphoryle,alkylalkoxy(thio)phosphoryle ou dialkoxy(thio)phosphoryle.

2. Nitroalcènes substitués de formule générale (I) suivant la revendication 1, dans laquelle
m représente le nombre 0, 1 ou 2,
A est un groupe alcanediyle en C₂ à C₅,
R¹ est un groupement hétérocyclique pentagonal ou hexagonal de la série furyle, thiényle, pyrrolyle, pyrazolyle, imidazolyle, 1,2,3 - ou 1,2,4-triazolyle, oxazolyle, isoxazolyle, 1,2,4- ou 1,3,4-oxadiazolyle, thiazolyle, isothiazolyle, 1,2,3-, 1,2,4-, 1,2,5- ou 1,3,4-thiadiazolyle, pyridyle, pyridazinyle, pyrimidinyle, pyrazinyle, substitué le cas échéant par du fluor, du chlore, du brome, de l'iode, un radical cyano, nitro, alkyle en C₁ à C₄ (qui est substitué le cas échéant par du fluor et/ou du chlore), alcényle en C₂ à C₄ (qui est substitué le cas échéant par du fluor et/ou du chlore), alcynyle en C₂ à C₄, alkoxy en C₁ à C₄ (qui est substitué le cas échéant par du fluor et/ou du chlore), alcényloxy en C₃ ou C₄ (qui est substitué le cas échéant par du fluor et/ou du chlore), alcynyloxy en C₃ ou C₄, alkylthio en C₁ à C₄ (qui est substitué le cas échéant par du fluor et/ou du chlore), alcénylthio en C₃ ou C₄ (qui est substitué le cas échéant par du fluor et/ou du chlore), alcynylthio en C₃ ou C₄, alkylsulfinyle en C₁ à C₄ (qui est substitué le cas échéant par du fluor et/ou du chlore), alkylsulfonyle en C₁ à C₄ (qui est substitué le cas échéant par du fluor et/ou du chlore), amino, alkylamino en C₁ à C₄, di-(alkyle en C₁ à C₄) - amino, phényle, phénoxy, phénylthio, phénylamino, benzyle, formylamino, (alkyle en C₁ à C₄)carbonylamino, formyle, carbamoyle, (alkyle en C₁ à C₄)carbonyle et/ou (alkoxy en C₁ à C₄)carbonyle,
R² représente l'hydrogène ou un groupe alkyle en C₁ à C₃,
R³ est l'hydrogène, un groupe alkyle en C₁ à C₄ (qui est substitué le cas échéant par un radical fluoro, chloro, cyano, alkoxy en C₁ à C₄ ou alkylthio en C₁ à C₄), un groupe alcényle en C₂ à C₄ (qui est substitué le cas échéant par du fluor, du chlore ou un radical phényle), un groupe alcynyle en C₂ à C₄, phényle (qui est substitué le cas échéant par du fluor, du chlore, du brome, un radical cyano, nitro, alkyle en C₁ à C₄, trifluorométhyle, alkoxy en C₁ à C₄, fluoralkoxy en C₁ ou C₂, chlorofluoralkoxy en C₁ ou C₂, alkylthio en C₁ à C₄, fluoralkylthio en C₁ ou C₂, chlorofluoralkylthio en C₁ ou C₂, alkylsulfinyle en C₁ à C₄, alkylsulfonyle en C₁ à C₄, fluoralkylsulfonyle en C₁ ou C₂, chlorofluoralkylsulfonyle en C₁ ou C₂, di-(alkyle en C₁ à C₃)amino ou (alkoxy en C₁ à C₃)carbonyle, un groupe benzyle (qui est substitué le cas échéant par du fluor, du chlore, un radical cyano, nitro, alkyle en C₁ ou C₂, trifluorométhyle ou (alkoxy en C₁ à C₃)-carbonyle), un groupe furyle, furylméthyle, thényle, thiényle ou pyridyle (qui sont substitués le cas échéant par du fluor, du chlore ou un radical alkyle en C₁ à C₄),
R⁴ est un groupe phényle (qui est substitué le cas échéant par du fluor, du chlore, du brome, un radical cyano, nitro, alkyle en C₁ à C₄ (qui est substitué le cas échéant par du fluor et/ou du chlore), un radical alkoxy en C₁ à C₄ (qui est substitué le cas échéant par du fluor et/ou du chlore), un radical alkylthio en C₁ à C₄ (qui est substitué le cas échéant par du fluor et/ou du chlore), un radical amino, alkylamino en C₁ à C₄, di(alkyle en C₁ à C₄)-amino ou (alkoxy en C₁ à C₄)carbonyle)), un groupe naphtyle (qui est substitué le cas échéant par du chlore, un radical cyano, nitro, amino ou alkyle en C₁ à C₄), un groupe pyridinyle (qui est substitué le cas échéant par du fluor, du chlore, du brome ou un radical alkyle en C₁ à C₄), un groupe pyrimidinyle (qui est substitué le cas échéant par du fluor, du chlore ou un radical alkyle en C₁ à C₄), un groupe imidazolyle ou triazolyle et
X représente l'oxygène, le soufre ou le groupement N-R⁵, dans lequel
R⁵ est l'hydrogène, un groupe alkyle en C₁ à C₄ (qui est substitué le cas échéant par du fluor, du chlore, un radical cyano, alkoxy en C₁ à C₄, alkylthio en C₁ à C₄, di(alkyle en C₁ à C₄)amino, triméthylsilyle, (alkoxy en C₁ à C₄)carbonyle, carboxy, carbamoyle, (alkyle en C₁ à C₄)aminocarbonyle, di(alkyle en C₁ à C₃)aminocarbonyle ou par le reste R¹, un groupe alcényle en C₂ à C₄ (qui est substitué le cas échéant par du fluor ou du chlore), un groupe alcynyle en C₂ à C₄, benzyle (qui est substitué le cas échéant par du fluor, du chlore, un radical cyano, nitro, alkyle en C₁ ou C₂, trifluorométhyle, alkoxy en C₁ ou C₂ ou (alkoxy en C₁ ou C₂)carbonyle), un groupe formyle, (alkyle en C₁ à C₂₀)carbonyle (qui est substitué le cas échéant par du fluor, du chlore, du brome, un radical cyano, phényle, phénoxy ou alkoxy en C₁ à C₄), un groupe (cycloalkyle en C₃ à C₆)carbonyle (qui est substitué le cas échéant par du fluor, du chlore et/ou un radical alkyle en C₁ à C₄), un groupe (alcényle en C₂ à C₂₀)carbonyle (qui est substitué le cas échéant par du fluor et/ou du chlore), un groupe phénylcarbonyle ou un groupe naphtylcarbonyle (qui sont substitués le cas échéant par du fluor, du chlore, du brome, un radical alkyle en C₁ à C₄, trifluorométhyle, cyano, nitro, alkoxy en C₁ à C₄ et/ou (alkoxy en C₁ à C₄)carbonyle, un groupe (alkoxy en C₁ à C₂₀)carbonyle, benzyloxycarbonyle, phénoxycarbonyle, (alkyle en C₁ à C₄)thiocarbonyle, benzylthiocarbonyle, phénylthiocarbonyle, (alkyle en C₁ à C₆)aminocarbonyle, di(alkyle en C₁ à C₄)aminocarbonyle, phénylaminocarbonyle (qui est substitué le cas échéant par du fluor, du chlore, du brome, un radical cyano, nitro, alkyle en C₁ à C₄, trifluorométhyle, alkoxy en C₁ à C₄, fluoralkoxy en C₁ ou C₂, chlorofluoralkoxy en C₁ ou C₂, alkylthio en C₁ à C₄, fluoralkylthio en C₁ ou C₂, chlorofluoralkylthio en C₁ ou C₂ ou (alkoxy en C₁, à C₄)carbonyle, un groupe benzoylaminocarbonyle (qui est substitué le cas échéant par du fluor, du chlore, du brome, un radical méthyle ou trifluorométhyle), un groupe phénylsulfonylaminocarbonyle (qui est substitué le cas échéant par du fluor, du chlore, du brome, un radical méthyle, trifluorométhyle, alkoxy en C₁ à C₄, fluoralkoxy en C₁ ou C₂, chlorofluoralkoxy en C₁ ou C₂ ou (alkoxy en C₁ à C₄)carbonyle), un groupe alkylthio en C₁ à C₄ (qui est substitué le cas échéant par du fluor et/ou du chlore), un groupe phénylthio (qui est substitué le cas échéant par du fluor, du chlore, du brome, un radical nitro ou méthyle, un groupe alkyl-sulfinyle en C₁ à C₄, alkylsulfonyle en C₁ à C₄ (qui est substitué le cas échéant par du fluor et/ou du chlore), un groupe phénylsulfinyle (qui est substitué le cas échéant par du fluor, du chlore, du brome, un radical nitro ou méthyle), un groupe phénylsulfonyle ou un groupe naphtylsulfonyle (qui sont substitués le cas échéant par du fluor, du chlore, du brome, un radical cyano, nitro, méthyle, trifluorométhyle, alkoxy en C₁ à C₄, fluoralkoxy en C₁ ou C₂, chlorofluoralkoxy en C₁ ou C₂ et/ou (alkoxy en C₁ à C₄)carbonyle), un groupe diméthyl(thio)phosphoryle, (alkyle en C₁ à C₄)-(alkoxy en C₁ à C₄)-(thio)phosphoryle ou di-(alkoxy en C₁ à C₄)-(thio)phosphoryle.

3. Nitroalcènes substitués de formule générale (I) suivant la revendication 1, dans laquelle
m représente le nombre 0, 1 ou 2,
A est un groupe éthane-1,2-diyle-(diméthylène) ou propane-1,3-diyle (triméthylène),
R¹ est un groupement hétérocyclique pentagonal ou hexagonal de la série pyrazolyle, 1,2,4-triazolyle, oxazolyle, isoxazolyle, thiazolyle, isothiazolyle, 1,2,5-thiadiazolyle, pyridyle, pyrazinyle et pyrimidinyle, qui est substitué le cas échéant par du fluor, du chlore, du brome, un radical cyano, nitro, alkyle en C₁ ou C₂ (qui est substitué le cas échéant par du fluor et/ou du chlore, alkoxy en C₁ ou C₂ (qui est substitué le cas échéant par du fluor et/ou du chlore), alkylthio en C₁ ou C₂ (qui est substitué le cas échéant par du fluor et/ou du chlore ou (alkyle en C₁ ou C₂)sulfonyle (qui est substitué le cas échéant par du fluor et/ou du chlore,
R² est l'hydrogène,
R³ est l'hydrogène,
R⁴ est un groupe phényle (qui est substitué le cas échéant par du fluor, du chlore, un radical alkyle en C₁ à C₄, trifluorométhyle, alkoxy en C₁ ou C₂ (qui est substitué le cas échéant par du fluor et/ou du chlore), ou (alkoxy en C₁ ou C₂)carbonyle, ou un groupe naphtyle et
X représente du soufre ou le groupement N-R⁵, dans lequel
R⁵ représente l'hydrogène, un groupe méthyle, éthyle, allyle, propargyle, formyle, (alkyle en C₁ à C₈)carbonyle, phénylcarbonyle (qui est substitué le cas échéant par du fluor et/ou du chlore), un groupe (alkoxy en C₁ à C₈)carbonyle, benzyloxycarbonyle, phénoxycarbonyle, benzyle (qui est substitué le cas échéant par du fluor ou du chlore), ou un groupe di-(alkoxy en C₁ ou C₂)-(thio)phosphoryle.

4. Procédé de production de nitroalcènes substitués de formule générale (I) 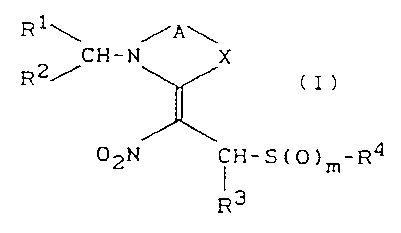 dans laquelle
m représente le nombre 0, 1 ou 2,
A est un groupe alcanediyle,
R¹ est un groupement hétérocyclique pentagonal ou hexagonal qui comporte 1, 2, 3 ou 4 atomes d'azote et/ou un ou deux atomes d'oxygène ou de soufre comme termes hétéro-atomiques du noyau - le nombre d'hétéro-atomes s'élevant à 1, 2, 3 ou 4 - et qui est substitué le cas échéant par un halogène, un groupe cyano, nitro, alkyle, halogénalkyle, alcényle, halogénalcényle, alcynyle, alkoxy, halogénalkoxy, alcényloxy, halogénalcényloxy, alcynyloxy, alkylthio, halogénalkylthio, alcénylthio, halogénalcénylthio, alcynylthio, alkylsulfinyle, halogénalkylsulfinyle, alkylsulfonyle, halogénalkylsulfonyle, amino, alkylamino, dialkylamino, aryle, aryloxy, arylthio, arylamino, aralkyle, formylamino, alkylcarbonylamino, formyle, carbamoyle, alkylcarbonyle et/ou alkoxycarbonyle,
R² est l'hydrogène ou un groupe alkyle,
R³ est l'hydrogène, un groupe alkyle (qui est substitué le cas échéant par un halogène, un radical cyano, alkoxy ou alkylthio), un groupe alcényle (qui est substitué le cas échéant par un halogène ou un radical phényle), un groupe alcynyle, phényle (qui est substitué le cas échéant par un halogène, un radical cyano, nitro, alkyle, halogénalkyle, alkoxy, halogénalkoxy, alkylthio, halogénalkylthio, alkylsulfinyle, alkylsulfonyle, halogénalkylsulfonyle, dialkylamino ou alkoxycarbonyle), un groupe benzyle (qui est substitué le cas échéant par un halogène, un radical cyano, nitro, alkyle, halogénalkyle ou alkoxycarbonyle), un groupe furyle, furylméthyle, thényle, thiényle ou pyridyle (chacun étant substitué le cas échéant par un halogène ou un radical alkyle),
R⁴ est un groupe phényle (qui est substitué le cas échéant par un halogène, un radical cyano, nitro, alkyle, halogénalkyle, alkoxy, halogénalkoxy, alkylthio, halogénalkylthio, amino, alkylamino, dialkylamino ou alkoxycarbonyle), un groupe naphtyle (qui est substitué le cas échéant par un halogène, un radical cyano, nitro, alkyle ou amino), un groupe pyridinyle (qui est substitué le cas échéant par un halogène ou un radical alkyle), un groupe pyrimidinyle (qui est substitué le cas échéant par un halogène ou un radical alkyle), un groupe imidazolyle ou triazolyle et
X représente l'oxygène, le soufre ou le groupement N-R⁵, dans lequel
R⁵ représente l'hydrogène, un groupe alkyle (qui est substitué le cas échéant par un halogène, un radical cyano, alkoxy, alkylthio, dialkylamino, trialkylsilyle, alkoxycarbonyle, carboxy, carbamoyle, alkylaminocarbonyle, dialkylaminocarbonyle ou par le reste R¹, lequel a la définition indiquée ci-dessus), un groupe alcényle (qui est substitué le cas échéant par un halogène), un groupe alcynyle, benzyle (qui est substitué le cas échéant par un halogène, un radical cyano, nitro, alkyle, halogénalkyle, alkoxy ou alkoxycarbonyle), un groupe formyle, alkylcarbonyle (qui est substitué le cas échéant par un halogène, un radical cyano, phényle, phénoxy ou alkoxy), un groupe cycloalkylcarbonyle (qui est substitué le cas échéant par un halogène et/ou un radical alkyle), un groupe alcénylcarbonyle (qui est substitué le cas échéant par un halogène), un groupe phénylcarbonyle ou naphtylcarbonyle (qui est substitué le cas échéant par un halogène, un radical alkyle, halogénalkyle, cyano, nitro, alkoxy et/ou alkoxycarbonyle), un groupe alkoxycarbonyle, benzyloxycarbonyle, phénoxycarbonyle, alkylthiocarbonyle, benzylthiocarbonyle, phénylthiocarbonyle, alkylaminocarbonyle, dialkylaminocarbonyle, phénylaminocarbonyle (qui est substitué le cas échéant par un halogène, un radical cyano, nitro, alkyle, halogénalkyle, alkoxy, halogénalkoxy, alkylthio, halogénalkylthio ou alkoxycarbonyle), un groupe benzoylaminocarbonyle (qui est substitué le cas échéant par un halogène), un radical alkyle ou halogénalkyle), un groupe phénylsulfonylaminocarbonyle (qui est substitué le cas échéant par un halogène, un radical alkyle, halogénalkyle, alkoxy, halogénalkoxy ou alkoxycarbonyle), un groupe alkylthio (qui est substitué le cas échéant par un halogène) un groupe phénylthio (qui est substitué le cas échéant par un halogène, un radical nitro ou alkyle), un groupe alkylsulfinyle, alkylsulfonyle (qui est substitué le cas échéant par un halogène), un groupe phénylsulfinyle (qui est substitué le cas échéant par un halogène, un radical nitro ou alkyle), un groupe phénylsulfonyle ou un groupe naphtylsulfonyle (qui sont substitués le cas échéant par un halogène, un radical cyano, nitro, alkyle, halogénalkyle, alkoxy, halogénalkoxy et/ou alkoxycarbonyle), un groupe dialkyl(thio)phosphoryle,alkylalkoxy(thio)phosphoryle ou dialkoxy(thio)phosphoryle,
caractérisé en ce que
a) pour l'obtention de composés de formule (I) dans laquelle A, R¹, R², R³, R⁴, X et m ont la définition indiquée ci-dessus,
on fait réagir des nitroalcènes de formule générale (II) 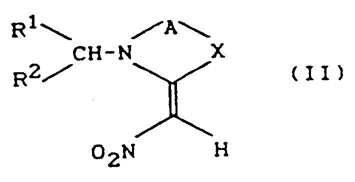 dans laquelle
A, R¹, R² et X ont les définitions indiquées ci-dessus,
- ou respectivement les isomères de configuration correspondante (Z et respectivement E) ou des mélanges des isomères Z et E -
avec des aldéhydes de formule générale (III) 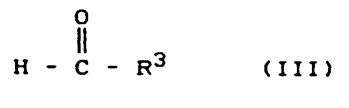 dans laquelle
R³ a la définition indiquée ci-dessus,
et avec des composés à groupe mercapto de formule générale (IV)
HS - R⁴ (IV)
dans laquelle
R⁴ a la définition indiquée ci-dessus,
en présence d'un acide et, le cas échéant, en présence d'un diluant, et on transforme éventuellement les composés ainsi obtenus de formule (I) dans laquelle m représente le nombre 0 et A, R¹, R², R³, R⁴ et X ont les définitions indiquées ci-dessus, par des procédés classiques d'oxydation en composés correspondants de formule (I) dans laquelle m représente le nombre 1 ou 2 et A, R¹, R², R³, R⁴ et X ont les définitions indiquées ci-dessus,
ou bien
b) pour obtenir des composés de formule (I) dans laquelle A, R¹, R², R³, R⁴ et m ont la définition indiquée ci-dessus et X représente le groupement NR⁵ dans lequel R⁵ a la définition indiquée pour la formule (I),
on fait réagir des nitroalcènes de formule (Ia) 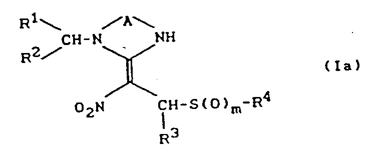 avec des composés halogénés de formule (VII)
Z-R⁵ (VII)
dans laquelle
R⁵ a la définition indiquée ci-dessus et
Z représente un halogène,
le cas échéant en présence d'un accepteur d'acide et en la présence éventuelle d'un diluant, à des températures comprises etre 10 et 100°C,
ou bien
c) pour l'obtention de composés de formule (I) dans laquelle A, R¹, R², R³, R⁴ et m ont la définition indiquée ci-dessus,
on fait réagir des amines de formule (VIII) 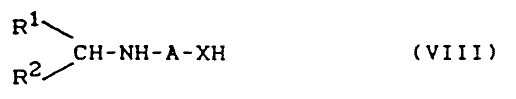 dans laquelle
R¹, R², A et X ont la définition indiquée ci-dessus,
avec des 1,1-bisméthylthio-3-nitro-alcènes de formule (IX) 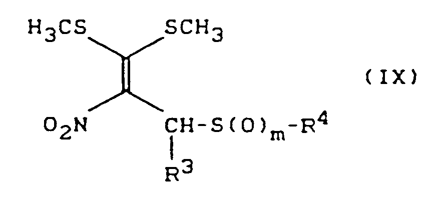 dans laquelle
R³, R⁴ et m ont la définition indiquée ci-dessus
le cas échéant en présence d'un diluant, à des températures comprises entre 10 et 150°C, ou bien
d) on fait réagir des composés halogénés de formule V 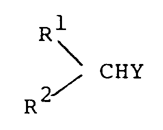 dans laquelle
R¹, R² ont la définition indiquée ci-dessus et Y représente un halogène,
avec des nitroalcènes de formule VI 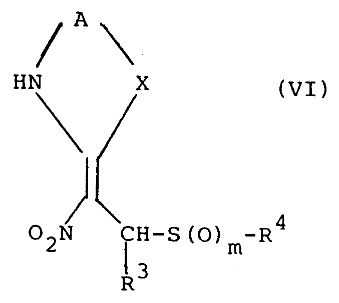 dans laquelle
A, R³, R⁴, X, m ont la définition indiquée ci-dessus,
le cas échéant en présence d'un accepteur d'acide et en la présence éventuelle d'un diluant, à des températures comprises entre 10 et 100°C.

5. Compositions pesticides, caractérisées par une teneur en au moins un nitroalcène substitué de formule (I).

6. Compositions insecticides suivant la revendication 5, caractérisées par une teneur en au moins un nitroalcène substitué de formule (I).

7. Procédé pour combattre des insectes, caractérisé en ce qu'on fait agir des nitroalcènes substitués de formule (I) sur des insectes et/ou sur leur milieu.

8. Utilisation de nitroalcènes substitués de formule (I) pour combattre des insectes.

9. Procédé de préparation de compositions pesticides, caractérisé en ce qu'on mélange des nitroalcènes substitués de formule (I) avec des diluants et/ou des agents tensio-actifs.
